# EUROPEAN PATENT APPLICATION

(11) **EP 1 715 044 A2**
(43) Date of publication of application: **25.10.2006**
(21) Application number: 06007573.6
(22) Date of filing: 26.01.2001
(51) Int. Cl.: C12N 15/12, C12N 15/11, C12N 15/62, C07K 14/705, C07K 16/28, C12Q 1/68, A61K 38/00

(54) **Human Neurexin-like proteins and polynucleotides encoding the same**

(30) Priority: 26.01.2000 US 178557 P; 25.04.2000 US 199513 P
(62) Divisional of application: 01926593.3
(71) Applicant: Lexicon Genetics Incorporated, The Woodlands, TX 77381-1160 (US)
(72) Inventor: Turner, Alexander C., Jnr., The Woodlands, TX 77382 (US); Hilbun, Erin, Denton, TX 76209 (US); Donoho, Gregory, Indianapolis, IN 46259 (US); Scoville, John, Pearland, TX 77584 (US); Wattler, Frank, 82541 Muensing (DE); Friedrich, Glenn, Houston, TX 77004 (US); Abuin, Alejandro, The Woodlands, TX 77382 (US); Zambrovicz, Brian, The Woodlands, TX 77382 (US); Sands, Arthur T., The Woodlands, TX 77382 (US)
(74) Representative: Hewson, Timothy John

(57) **Abstract**

Novel human polynucleotide and polypeptide sequences are disclosed that can be used in therapeutic, diagnostic, and pharmacogenomic applications.

## Description

The present application claims benefit of priority to U.S. Applications Ser. Nos. 60/178,557, filed January 26, 2000, and 60/199,513, filed April 25, 2000 which are herein incorporated by reference in their entirety.

### 1. INTRODUCTION

The present invention relates to the discovery, identification, and characterization of novel human polynucleotides encoding proteins that share sequence similarity with animal neurexin proteins and contactin associated proteins. The invention encompasses the described polynucleotides, host cell expression systems, the encoded proteins, fusion proteins, polypeptides and peptides, antibodies to the encoded proteins and peptides, and genetically engineered animals that either lack or over express the disclosed sequences, antagonists and agonists of the proteins, and other compounds that modulate the expression or activity of the proteins encoded by the disclosed sequences that can be used for diagnosis, drug screening, clinical trial monitoring, the treatment of diseases and disorders, or cosmetic or nutriceutical applications.

### 2. BACKGROUND OF THE INVENTION

Neurexins have been associated with, *inter alia*, mediating neural processes, seizures, signaling, exocytosis, cancer, and development. Neurexins can also serve as receptors for latrotoxins.

### 3. SUMMARY OF THE INVENTION

The present invention relates to the discovery, identification, and characterization of nucleotides that encode novel human proteins and the corresponding amino acid sequences of these proteins. The novel human proteins (NHPs) described for the first time herein share structural similarity with neurexin proteins.

The novel human nucleic acid sequences described herein, encode alternative proteins/open reading frames (ORFs) of 1,307, 1,259, 35, 250, 279, 582, 534, 745, 697, 839, 791, 1,298, and 1,175 amino acids in length (see respecrively SEQ ID NOS: 2, 4, 6, 8, 10, 12, 14, 16, 18, 20, 22, 24, and 26).

The invention also encompasses agonists and antagonists of the described NHPs, including small molecules, large molecules, mutant NHPs, or portions thereof that compete with native NHP, peptides, and antibodies, as well as nucleotide sequences that can be used to inhibit the expression of the described NHPs (e.g., antisense and ribozyme molecules, and gene or regulatory sequence replacement constructs) or to enhance the expression of the described NHP sequences (e.g., expression constructs that place the described sequence under the control of a strong promoter system), and transgenic animals that express a NHP transgene, or "knockouts" (which can be conditional) that do not express a functional NHP.

Further, the present invention also relates to processes for identifying compounds that modulate, i.e., act as agonists or antagonists, of NHP expression and/or NHP activity that utilize purified preparations of the described NHPs and/or NHP product, or cells expressing the same. Such compounds can be used as therapeutic agents for the treatment of any of a wide variety of symptoms associated with biological disorders or imbalances.

### 4. DESCRIPTION OF THE SEQUENCE LISTING AND FIGURES

The Sequence Listing provides the sequences of the described NHP ORFs that encode the described NHP amino acid sequences. SEQ ID NO:27 describes a NHP ORF and flanking regions.

### 5. DETAILED DESCRIPTION OF THE INVENTION

The NHPs, described for the first time herein, are novel proteins that are expressed in, *inter alia,* human cell lines, human fetal brain, brain, cerebellum, testis, adrenal gland, spinal cord, small intestine, hypothalamus, and gene trapped human cells.

The present invention encompasses the nucleotides presented in the Sequence Listing, host cells expressing such nucleotides, the expression products of such nucleotides, and: (a) nucleotides that encode mammalian homologs of the described sequences, including the described NHPs, and the NHP products; (b) nucleotides that encode one or more portions of the NHPs that correspond to functional domains, and the polypeptide products specified by such nucleotide sequences, including but not limited to the novel regions of any active domain(s); (c) isolated nucleotides that encode mutant versions, engineered or naturally occurring, of the described NHPs in which all or a part of at least one domain is deleted or altered, and the polypeptide products specified by such nucleotide sequences, including but not limited to soluble proteins and peptides in which all or a portion of the signal sequence is deleted; (d) nucleotides that encode chimeric fusion proteins containing all or a portion of a coding region of a NHP, or one of its domains (e.g., a receptor or ligand binding domain, accessory protein/self-association domain, etc.) fused to another peptide or polypeptide; or (e) therapeutic or diagnostic derivatives of the described polynucleotides such as oligonucleotides, antisense polynucleotides, ribozymes, dsRNA, or gene therapy constructs comprising a sequence first disclosed in the Sequence Listing.

As discussed above, the present invention includes: (a) the human DNA sequences presented in the Sequence Listing (and vectors comprising the same) and additionally contemplates any nucleotide sequence encoding a contiguous NHP open reading frame (ORF) that hybridizes to a complement of a DNA sequence presented in the Sequence Listing under highly stringent conditions, e.g., hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. *et al*., eds., 1989, Current Protocols in Molecular Biology, Vol. I, Green Publishing Associates, Inc., and John Wiley & sons, inc., New York, at p. 2.10.3) and encodes a functionally equivalent gene product. Additionally contemplated are any nucleotide sequences that hybridize to the complement of a DNA sequence that encodes and expresses an amino acid sequence presented in the Sequence Listing under moderately stringent conditions, *e.g.,* washing in 0.2xSSC/0.1% SDS at 42°C (Ausubel et al., 1989, *supra*), yet still encodes a functionally equivalent NHP product. Functional equivalents of a NHP include naturally occurring NHPs present in other species and mutant NHPs whether naturally occurring or engineered (by site directed mutagenesis, gene shuffling, directed evolution as described in, for example, U.S. Patents Nos. 5,837,458 and 5,723,323 both of which are herein incorporated by reference in their entiraty). The invention also includes degenerate nucleic acid variants of the disclosed NHF polynucleotide sequences.

Additionally contemplated are polynucleotides encoding NHP ORFs, or their functional equivalents, encoded by polynucleotide sequences that are about 99, 95, 90, or about 85 percent similar or identical to corresponding regions of the nucleotide sequences of the Sequence Listing (as measured by BLAST sequence comparison analysis using, for example, the GCG sequence analysis package (Madison, Wisconsin) using standard default settings).

The invention also includes nucleic acid molecules, preferably DNA molecules, that hybridize to, and are therefore the complements of, the described NHP nucleotide sequences. Such hybridization conditions may be highly stringent or less highly stringent, as described above. In instances where the nucleic acid molecules are deoxyoligonucleotides ("DNA oligos"), such molecules are generally about 16 to about 100 bases long, or about 20 to about 80, or about 34 to about 45 bases long, or any variation or combination of sizes represented therein that incorporate a contiguous region of sequence first disclosed in the Sequence Listing. Such oligonucleotides can be used in conjunction with the polymerase chain reaction (PCR) to screen libraries, isolate clones, and prepare cloning and sequencing templates, etc.

Alternatively, such NHP oligonucleotides can be used as hybridization probes for screening libraries, and assessing gene expression patterns (particularly using a micro array or high-throughput "chip" format). Additionally, a series of the described NHP oligonucleotide sequences, or the complements thereof, can be used to represent all or a portion of the described NHP sequences. An oligonucleotide or polynucleotide sequence first disclosed in at least a portion of one or more of the sequences of SEQ ID NOS: 1-27 can be used as a hybridization probe in conjunction with a solid support matrix/substrate (resins, beads, membranes, plastics, polymers, metal or metallized substrates, crystalline or polycrystalline substrates, etc.). Of particular note are spatially addressable arrays (i.e., gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one of the sequences of SEQ ID NOS: 1-27, or an amino acid sequence encoded thereby. Methods for attaching biopolymers to, or synthesizing biopolymers on, solid support matrices, and conducting binding studies thereon are disclosed in, *inter alia,* U.S. Patent Nos. 5,700,637, 5,556,752, 5,744,305, 4,631,211, 5,445,934, 5,252,743, 4,713,326, 5,424,186, and 4,689,405 the disclosures of which are herein incorporated by reference in their entirety.

Addressable arrays comprising sequences first disclosed in SEQ ID NOS:1-27 can be used to identify and characterize the temporal and tissue specific expression of a gene. These addressable arrays incorporate oligonucleotide sequences of sufficient length to confer the required specificity, yet be within the limitations of the production technology. The length of these probes is within a range of between about 8 to about 2000 nucleotides. Preferably the probes consist of 60 nucleotides and more preferably 25 nucleotides from the sequences first disclosed in SEQ ID NOS:1-27.

For example, a series of the described oligonucleotide sequences, or the complements thereof, can be used in chip format to represent all or a portion of the described sequences. The oligonucleotides, typically between about 16 to about 40 (or any whole number within the stated range) nucleotides in length can partially overlap each other and/or the sequence may be represented using oligonucleotides that do not overlap. Accordingly, the described polynucleotide sequences shall typically comprise at least about two or three distinct oligonucleotide sequences of at least about 8 nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense orientation.

Microarray-based analysis allows the discovery of broad patterns of genetic activity, providing new understanding of gene functions and generating novel and unexpected insight into transcriptional processes and biological mechanisms. The use of addressable arrays comprising sequences first disclosed in SEQ ID NOS:1-27 provides detailed information about transcriptional changes involved in a specific pathway, potentially leading to the identification of novel components or gene functions that manifest themselves as novel phenotypes.

Probes consisting of sequences first disclosed in SEQ ID NOS:1-27 can also be used in the identification, selection and validation of novel molecular targets for drug discovery. The use of these unique sequences permits the direct confirmation of drug targets and recognition of drug dependent changes in gene expression that are modulated through pathways distinct from the drugs intended target. These unique sequences therefore also have utility in defining and monitoring both drug action and toxicity.

As an example of utility, the sequences first disclosed in SEQ ID NOS:1-27 can be utilized in microarrays or other assay formats, to screen collections of genetic material from patients who have a particular medical condition. These investigations can also be carried out using the sequences first disclosed in SEQ ID NOS:1-27 *in silico* and by comparing previously collected genetic databases and the disclosed sequences using computer software known to those in the art.

Thus the sequences first disclosed in SEQ ID NOS:1-27 can be used to identify mutations associated with a particular disease and also as a diagnostic or prognostic assay.

Although the presently described sequences have been specifically described using nucleotide sequence, it should be appreciated that each of the sequences can uniquely be described using any of a wide variety of additional structural attributes, or combinations thereof. For example, a given sequence can be described by the net composition of the nucleotides present within a given region of the sequence in conjunction with the presence of one or more specific oligonucleotide sequence(s) first disclosed in the SEQ ID NOS: 1-27. Alternatively, a restriction map specifying the relative positions of restriction endonuclease digestion sites, or various palindromic or other specific oligonucleotide sequences can be used to structurally describe a given sequence. Such restriction maps, which are typically generated by widely available computer programs (e.g., the University of Wisconsin GCG sequence analysis package, SEQUENCHER 3.0, Gene Codes Corp., Ann Arbor, MI, etc.), can optionally be used in conjunction with one or more discrete nucleotide sequence(s) present in the sequence that can be described by the relative position of the sequence relatve to one or more additional sequence(s) or one or more restriction sites present in the disclosed sequence.

For oligonucleotide probes, highly stringent conditions may *refer, e*.*g*., to washing in 6xSSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NHP gene antisense molecules, useful, for example, in NHP gene regulation (for and/or as antisense primers in amplification reactions of NHP nucleic acid sequences). With respect to NHP gene regulation, such techniques can be used to regulate biological functions. Further, such sequences may be used as part of ribozyme and/or triple helix sequences that are also useful for NHP gene regulation.

Inhibitory antisense or double stranded oligonucleotides can additionally comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcyrosine, N6-adenine, 7-methylguanine, 5-methylaminomethyluracil, 5-methoxyaminomethyl-2-thiouracil, beta-D-manncsylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar moiety selected from the group including but not limited to arabinose, 2-fluoroarabinose, xylulose, and hexose.

In yet another embodiment, the antisense oligonucleotide will comprise at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al*., 1987, Nucl. Acids Res. *15*:6625-6641). The oligonucleotide is a 2'-0-methylribonucleotide (Inoue *et al*., 1987, Nucl. Acids Res. *15*:6131-6148), or a chimeric RNA-DNA analogue (Inoue *et al*., 1987, FEBS Lett. *215*:327-330). Alternatively, double stranded RNA can be used to disrupt the expression and function of a targeted NHP.

Oligonucleotides of the invention can be synthesized by standard methods known in the art, e.g. by use of an automated DNA synthesizer (such as are commercially available from Biosearch, Applied Biosystems, etc.). As examples, phosphorothicate oligonucleotides can be synthesized by the method of Stein *et al*. (1988, Nucl. Acids Res. *16*:3209), and methylphosphonate oligonucleotides can be prepared by use of controlled pore glass polymer supports (Sarin *et al.,* 1988, Proc. Natl. Acad. Sci. U.S.A. *85*:7448-7451), etc.

Low stringency conditions are well known to those of skill in the art, and will vary predictably depending on the specific organisms from which the library and the labeled sequences are derived. For guidance regarding such conditions see, for example, Sambrook *et al*., 1989, Molecular Cloning, A Laboratory Manual (and periodic updates thereof), Cold Springs Harbor Press, N.Y.; and Ausubel *et al*., 1989, Current Protocols in Molecular Biology, Green Publishing Associates and Wiley Interscience, N.Y.

Alternatively, suitably labeled NHP nucleotide probes can be used to screen a human genomic library using appropriately stringent conditions or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (e.g., splice acceptor and/or donor sites), etc., that can be used in diagnostics and pharmacogenomics.

Further, a NHP gene homolog can be isolated from nucleic acid from an organism of interest by performing PCR using two degenerate or "wobble" oligonucleotide primer pools designed on the basis of amino acid sequences within the NHP products disclosed herein. The template for the reaction may be total RNA, mRNA, and/or cDNA obtained by reverse transcription of mRNA prepared from human or non-human cell lines or tissue known or suspected to express an allele of a NHP gene.

The PCR product can be subcloned and sequenced to ensure that the amplified sequences represent the sequence of the desired NHP gene. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library.

PCR technology can also be used to isolate full length cDNA sequences. For example, RNA can be isolated, following standard procedures, from an appropriate cellular or tissue source (*i.e*., one known, or suspected, to express a NHP sequence). A reverse transcription (RT) reaction can be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment can be isolated. For a review of cloning strategies that can be used, see *e.g*., Sambrook *et al.,* 1989, *supra.*

A cDNA encoding a mutant NHP sequence can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NHP allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well known to those of skill in the art. By comparing the DNA sequence of the mutant NHP allele to that of a corresponding normal NHP allele, the mutation(s) responsible for the loss or alteration of function of the mutant NHP gene product can be ascertained.

Alternatively, a genomic library can be constructed using DNA obtained from an individual suspected of or known to carry a mutant NHP allele (*e.g.,* a person manifesting a NHP-associated phenotype such as, for example, obesity, vision disorders, high blood pressure, depression, seizures, infertility, etc.), or a cDNA library can be constructed using RNA from a tissue known, or suspected, to express a mutant NHP allele. A normal NHP gene, or any suitable fragment thereof, can then be labeled and used as a probe to identify the corresponding mutant NHP allele in such libraries. Clones containing mutant NHP gene sequences can then be purified and subjected to sequence analysis according to methods well known to those skilled in the art.

Additionally, an expression library can be constructed utilizing cDNA synthesized from, for example, RNA isolated from a tissue known, or suspected, to express a mutant NHP allele in an individual suspected of or known to carry such a mutant allele. In this manner, gene products made by the putatively mutant tissue can be expressed and screened using standard antibody screening techniques in conjunction with antibodies raised against a normal NHP product, as described below. (For screening techniques, see, for example, Harlow, E. and Lane, eds., 1988, "Antibodies: A Laboratory Manual", Cold Spring Harbor Press, Cold Spring Harbor.)

Additionally, screening can be accomplished by screening with labeled NHP fusion proteins, such as, for example, alkaline phosphatase-NHP or NHP-alkaline phosphatase fusion proteins. In cases where a NHP mutation results in an expressed gene product with altered function (*e*.*g*., as a result of a missense or a frameshift mutation), polyclonal antibodies to a NHP are likely to cross-react with a corresponding mutant NHP gene product. Library clones detected via their reaction with such labeled antibodies can be purified and subjected to sequence analysis according to methods well known in the art.

The invention also encompasses (a) DNA vectors that contain any of the foregoing NHP coding sequences and/or their complements (*i.e.,* antisense); (b) DNA expression vectors that contain any of the foregoing NHP coding sequences operatively associated with a regulatory element that directs the expression of the coding sequences (for example, baculo virus as described in U.S. Patent No. 5,869,336 herein incorporated by reference); (c) genetically engineered host cells that contain any of the foregoing NHP coding sequences operatively associated with a regulatory element that directs the expression of the coding sequences in the host cell; and (d) genetically engineered host cells that express an endogenous NHP gene under the control of an exogenously introduced regulatory element (*i*.*e*., gene activation). As used herein, regulatory elements include, but are not limited to, inducible and non-inducible promoters, enhancers, operators and other elements known to those skilled in the art that drive and regulate expression. Such regulatory elements include but are not limited to the cytomegalovirus (hCMV) immediate early gene, regulatable, viral elements (particularly retroviral LTR promoters), the early or late promoters of SV40 adenovirus, the *lac* system, the *trp* system, the *TAC* system, the *TRC* system, the major operator and promoter regions of phage lambda, the control regions of fd coat protein, the promoter for 3-phosphoglycerate kinase (PGK), the promoters of acid phosphatase, and the promoters of the yeast α-mating factors.

The present invention also encompasses antibodies and anti-idiotypic antibodies (including Fab fragments), antagonists and agonists of the NHP, as well as compounds or nucleotide constructs that inhibit expression of a NHP gene (transcription factor inhibitors, antisense and ribozyme molecules, or gene or regulatory sequence replacement constructs), or promcte the expression of a NHP (e.g., expression constructs in which NHP coding sequences are operatively associated with expression control elements such as promoters, promoter/enhancers, etc.).

The NHPs or NHP peptides, NHP fusion proteins, NHP nucleotide sequences, antibodies, antagonists and agonists can be useful for the detection of mutant NHPs or inappropriately expressed NHPs for the diagnosis of disease. The NHP proteins or peptides, NHP fusion proteins, NHP nucleotide sequences, host cell expression systems, antibodies, antagonists, agonists and genetically engineered cells and animals can be used for screening for drugs (or high throughput screening of combinatorial libraries) effective in the treatment cf the symptomatic or phenotypic manifestations of perturbing the normal function of NHP in the body. The use of engineered host cells and/or animals may offer an advantage in that such systems allow not only for the identification of compounds that bind to the endogenous receptor for an NHP, but can also identify compounds that trigger NHP-mediated activities or pathways.

Finally, the NHP products can be used as therapeutics. For example, soluble derivatives such as NHP peptides/domains corresponding to the NHPs, secreted forms of a NHP, NHP fusion protein products (especially NHP-Ig fusion proteins, i.e., fusions of a NHP, or a domain of a NHP, to an IgFc), NHF antibodies and anti-idiotypic antibodies (including Fab fragments), antagonists or agonists (including compounds that modulate or act on downstream targets in a NHP-mediated pathway) can be used to directly treat diseases or disorders. For instance, the administration of an effective amount of soluble NHP, or a NHP-IgFc fusion protein or an anti-idiotypie antibody (or its Fab) that mimics the NHP could activate or effectively antagonize the endogenous NHP receptor. Nucleotide constructs encoding such NHP products can be used to genetically engineer host cells to express such products *in vivo;* these genetically engineered cells function as "bioreactors" in the body delivering a continuous supply of a NHP, a NHP peptide, or a NHP fusion protein to the body. Nucleotide constructs encoding functional NHPs, mutant NHPs, as well as antisense and ribozyme molecules can also be used in "gene therapy" approaches for the modulation of NHP expression. Thus, the invention also encompasses pharmaceutical formulations and methods for treating biological disorders.

Various aspects of the invention are described in greater detail in the subsections below.

### 5.1 THE NHP SEQUENCES

The cDNA sequences and the corresponding deduced amino acid sequences of the described NHPs are presented in the Sequence Listing. The NHP nucleotides were obtained from clustered human gene trapped sequences, ESTs, and cDNAs isolated from human brain, fetal brain, cerebellum, and hypothalamus cDNA libraries (Edge Biosystems, Gaithersburg, MD). The described sequences share limited structural similarity with a variety of proteins, including, but not limited to, neurexins (including secreted types) and contactin associated proteins. A polymorphism was identified that results in a C-or-T transition at, for example, the position corresponding to nucleotide 812 of SEQ ID NO:1 that can result in a ser or leu being present at, for example, amino acid position 271 of SEQ ID NO:2.

### 5.2 NHPS AND NHP POLYPEPTIDES

NHPs, polypeptides, peptide fragments, mutated, truncated, or deleted forms of the NHPs, and/or NHP fusion proteins can be prepared for a variety of uses. These uses include, but are not limited to, the generation of antibodies, as reagents in diagnostic assays, for the identification of other cellular gene products related to a NHP, as reagents in assays for screening for compounds that can be as pharmaceutical reagents useful in the therapeutic treatment of mental, biological, or medical disorders and disease. Given the similarity information and expression data, the described NHPs can be targeted (by drugs, oligos, antibodies, etc,) in order to treat disease, or to therapeutically augment the efficacy of therapeutic agents.

The Sequence Listing discloses the amino acid sequences encoded by the described NHP sequences. The NHPs typically display initiator methionines in DNA sequence contexts consistent with a translation initiation site, and signal sequences characteristic of membrane or secreted proteins.

The NHP amino acid sequences of the invention include the amino acid sequences presented in the Sequence Listing as well as analogues and derivatives thereof. Further, corresponding NHP homologues from other species are encompassed by the invention. In fact, any NHP protein encoded by the NHP nucleotide sequences described above are within the scope of the invention, as are any novel polynucleotide sequences encoding all or any novel portion of an amino acid sequence presented in the Sequence Listing. The degenerate nature of the genetic code is well known, and, accordingly, each amino acid presented in the Sequence Listing, is generically representative of the well known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell *et al*. eds., Scientific American Books, New York, NY, herein incorporated by reference) are generically representative of all the various permutations and combinations of nucleic acid sequences that can encode such amino acid sequences.

The invention also encompasses proteins that are functionally equivalent to the NHPs encoded by the presently described nucleotide sequences as judged by any of a number of criteria, including, but not limited to, the ability to bind and cleave a substrate of a NHP, or the ability to effect an identical or complementary downstream pathway, or a change in cellular metabolism (e.g., proteolytic activity, ion flux, tyrosine phosphorylation, transport, etc.). Such functionally equivalent NHP proteins include, but are not limited to, additions or substitutions of amino acid residues within the amino acid sequence encoded by the NHP nucleotide sequences described above, but which result in a silent change, thus producing a functionally equivalent gene product. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

A variety of host-expression vector systems can be used to express the NHP nucleotide sequences of the invention. Where, as in the present instance, the NHP peptides or polypeptides are thought to be membrane proteins, the hydrophobic regions of the protein can be excised at the protein or nucleic acid level (i.e., the hydrophobic region spanning from roughly about amino acid number 1,240 to about amino acid position 1,265 of, for example, SEQ ID NO:2) and the resulting soluble peptide or polypeptide can be recovered from the culture media. Such expression systems also encompass engineered host cells that express a NHP, or a functional equivalent, in situ. Purification or enrichment of a NHP from such expression systems can be accomplished using appropriate detergents and lipid micelles and methods well known to those skilled in the art. However, such engineered host cells themselves may be used in situations where it is important not only to retain the structural and functional characteristics of the NHP, but to assess biological activity, *e.g.,* in drug screening assays.

The expression systems that can be used for purposes of the invention include but are not limited to microorganisms such as bacteria (*e*.*g*., *E. coli, B. subtilis*) transformed with recombinant bacteriophage DNA, plasmid DNA or cosmid DNA expression vectors containing NHP nucleotide sequences; yeast (*e*.*g*., *Saccharomyces, Pichia*) transformed with recombinant yeast expression vectors containing NHP nucleotide sequences; insect cell systems infected with recombinant virus expression vectors (*e*.*g*., baculovirus) containing NHP sequences; plant cell systems infected with recombinant virus expression vectors (*e*.*g*., cauliflower mosaic virus, CaMV; tobacco mosaic virus, TMV) or transformed with recombinant plasmid expression vectors (e.g., Ti plasmid) containing NHP nucleotide sequences; or mammalian cell systems (e.g., COS, CHO, BHK, 293, 3T3) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of or containing NHP, cr for raising antibodies to a NHP, vectors that direct the expression of high levels of fusion protein products that are readily purified may be desirable. Such vectors include, but are not limited, to the *E*. *coli* expression vector pUR278 (Ruther *et al*., 1983, EMBO J. 2:1791), in which a NHP coding sequence may be ligated individually into the vector in frame with the *lacZ* coding region so that a fusion protein is produced; pIN vectors (Inouye & Inouye, 1985, Nucleic Acids Res. *13*:3101-3109; Van Heeke & Schuster, 1989, J. Biol. Chem. *264*:5503-5509); and the like. pGEX vectors (Pharmacia or American Type Culture Collection) can also be used to express foreign polypeptides as fusion proteins with glutathione S-transferuse (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, *Autographa californica* nuclear polyhidrosis virus (AcNPV) is used as a vector to express foreign genes. The virus grows in *Spodoptera frugiperda* cells. A NHP coding sequence may be cloned individually into non-essential regions (for example the polyhedrin gene) of the virus and placed under control of an AcNPV promoter (for example the polyhedrin prometer). Successful insertion of NHP coding sequence will result in inactivation of the polyhedrin gene and production of non-occluded recombinant virus (*i*.*e*., virus lacking the proieinaceous coat coded for by the polyhedrin gene). These recombinant viruses are then used to infect Spodoptera frugiperda cells in which the inserted sequence is expressed (*e*.*g*., see Smith *et al*., 1983, J. Virol. *46*:584; Smith, U.S. Patent No. 4,215,051).

In mammalian host cells, a number of viral-based expression systems may be utilized. In cases where an adenovirus is used as an expression vector, the NHP nucleotide sequence of interest may be ligated to an adenovirus transcription/translation control complex, e.g., the late promoter and tripartite leader sequence. This chimeric gene may then be inserted in the adenovirus genome by *in vitro* or *in vivo* recombination. Insertion in a non-essential region of the viral genome (*e.g*., region E1 or E3) will result in a recombinant virus that is viable and capable of expressing a NHP product in infected hosts (*e.g*., See Logan & Shenk, 1984, Proc. Natl. Acad. Sci. USA *81*:3655-3659). Specific initiation signals may also be required for efficient translation of inserted NHP nucleotide sequences. These signals include the ATG initiation codon and adjacent sequences. In cases where an entire NHP gene or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be cf a variety of origins, both natural and synthetic. The efficiency of expression may be enhanced by the inclusion of appropriate transcription enhancer elements, transcription terminators, etc. (See Bitter *et al*., 1987, Methods in Enzymol. *153*:516-544).

In addition, a host cell strain may be chosen that modulates the expression of the inserted sequences, or modifies and processes the gene product in the specific fashion desired. Such modifications (*e*.*g*., glycosylation) and processing (*e*.*g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines which stably express the NHP sequences described above can be engineered. Rather than using expression vectors which contain viral origins of replication, host cells can be transformed with DNA controlled by appropriate expression control elements (*e.g.,* promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form fcci which in turn can be cloned and expanded into cell lines. This method may advantageously be used to engineer cell lines which express the NHP product. Such engineered cell lines may be particularly useful in screening and evaluation of compounds that affect the endogenous activity of the NHP product.

A number of selection systems may be used, including but not limited to the herpes simplex virus thymidine kinase (Wigler, *et al*., 1977, Cell *11*:223), hypoxanthine-guanine phosphoribosyltransferase (Szybalska & Szybalski, 1962, Proc. Natl. Acad. Sci. USA *48*:2026), and adenine phosphoribosyltransferase (Lowy, *et al.,* 1980, Cell *22*:817) genes can be employed in tk⁻, hgprt⁻ or aprt⁻ cells, respectively. Also, antimetabolite resistance can be used as the basis of selection for the following genes: dhfr, which confers resistance to methotrexate (Wigler, *et al*., 1980, Natl. Acad. Sci. USA *77*:3567; O'Hare, *et al*., 1981, Proc. Natl. Acad. Sci. USA *78*:1527); gpt, which confers resistance to mycophenolic acid (Mulligan & Berg, 1981, Proc. Natl. Acad. Sci. USA *78*:2072); neo, which confers resistance to the aminoglyceside G-418 (Colberre-Garapin, *et al*., 1981, J. Mol. Biol. *150*:1); and hygro, which confers resistance to hygromycin (Santerre, *et al*., 1984, Gene *30*:147).

Alternatively, any fusion protein can be readily, purified by utilizing an antibody specific for the fusion protein being expressed. For example, a system described by Jsnknecht *et al*. allows for the ready purification of non-denatured fusion proteins expressed in human cell lines (Janknecht, *et al*., 1991, Proc. Natl. Acad. Sci. USA *88*-8972-8976). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺ nitriloacetic acid-agarose columns and histidine-tagged proteins are selectively eluted with imidazole-containing buffers.

Also encompassed by the present invention are fusion proteins that direct the NHP to a target organ and/or facilitate transport across the membrane into the cytosol. Conjugation of NHPs to antibody molecules or their Fab fragments could be used to target cells bearing a particular epitope. Attaching the appropriate signal sequence to the NHP would also transport the NHP to the desired location within the cell. Alternatively targeting of NHP or its nucleic acid sequence might be achieved using liposome or lipid complex based delivery systems. Such technologies are described in Liposomes:A Practical Approach, New,RRC ed., Oxford University Press, New York and in U.S. Patents Nos. 4,594,595, 5,459,127, 5,948,767 and 6,110,490 and their respective disclosures which are herein incorporated by reference in their entirety. Additionally embodied are novel protein constructs engineered in such a way that they facilitate transport of the NHP to the target site or desired organ, where they cross the cell membrane and/or the nucleus where the NHP can exert its functional activity. This goal may be achieved by coupling of the NHP to a cytokine or other ligand that provides targeting specificity, and/or to a protein transducing domain (see generally U.S. applications Ser. No. 60/111, 701 and 60/056,713, both of which are herein incorporated by reference, for examples of such transducing sequences) to facilitate passage across cellular membranes and can optionally be engineered to include nuclear localization sequences.

### 5.3 ANTIBODIES TO NHP PRODUCTS

Antibodies that specifically recognize one or more epitopes of a NHP, or epitopes of conserved variants of a NHP, or peptide fragments of a NHP are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of NHP in a biological sample and may, therefore, be utilized as part of a diagnostic or prognostic technique whereby patients may be tested for abnormal amounts of NHP. Such antibodies may also be utilized in conjunction with, for example, compound screening schemes for the evaluation of the effect of test compounds on expression and/or activity of a NHP gene product. Additionally, such antibodies can be used in conjunction gene therapy to, for example, evaluate the normal and/or engineered NHP-expressing cells prior to their introduction into the patient. Such antibodies may additionally be used as a method for the inhibition of abnormal NHP activity. Thus, such antibodies may, therefore, be utilized as part of treatment methods.

For the production of antibodies, various host animals may be immunized by injection with the NHP, an NHP peptide (*e.g*., one corresponding to a functional domain of an NHP), truncated NHP polypeptides (NHP in which one or more domains have been deleted), functional equivalents of the NHP or mutated variant of the NHP. Such host animals may include but are not limited to pigs, rabbits, mice, goats, and rats, to name but a few. Various adjuvants may be used to increase the immunological response, depending on the host species, including but not limited to Freund's adjuvant (complete and incomplete), mineral salts such as aluminum hydroxide or aluminum phosphate, surface active substances such as lysolecithin, pluronic polyols, polyanions, peptides, oil emulsions, and potentially useful human adjuvants such as BCG (bacille Calmette-Guerin) and *Corynebacterium parvum.* Alternatively, the immune response could be enhanced by combination and or coupling with molecules such as keyhole limpet hemocyanin, tetanus toxoid, diptheria toxoid, ovalbumin, cholera toxin or fragments thereof. Polyclonal antibodies are heterogeneous populations of antibody molecules derived from the sera of the immunized animals.

Monoclonal antibodies, which are homogeneous populations of antibodies to a particular antigen, can be obtained by any technique which provides for the production of antibody molecules by continuous cell lines in culture. These include, but are not limited to, the hybridoma technique of Kohler and Milstein, (1975, Nature *256*:495-497; and U.S. Patent No. 4,376,110), the human B-cell hybridoma technique (Kosbor *et al*., 1983, Immunology Today 4:72; Cole *et al*., 1983, Proc. Natl. Acad. Sci. USA *80*:2026-2030), and the EBV-hybridoma technique (Cole *et al*., 1985, Monoclonal Antibodies And Cancer Therapy, Alan R. Liss, Inc., pp. 77-96). Such antibodies may be cf any immunoglobulin class including IgG, IgM, IgE, IgA, IgD and any subclass thereof. The hybridoma producing the mAb of this invention may be cultivated in vitro or *in vivo*. Production of high titers of mAbs in vivo makes this the presently preferred method of production.

In addition, techniques developed for the production of "chimeric antibodies" (Morrison *et al.,* 1984, Proc. Natl. Acad. Sci., *81*:6851-6855; Neuberger *et al*., 1984, Nature, *312*:604-608; Takeda *et al*., 1985, Nature, *314*:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region (see U.S. Patents Nos. 6,075,181 and 5,877,397 both of which are herein incorporated by reference in their entirety). Also useful is the production of fully humanized monoclonal antibodies as described in US Patent No. 6,150,584 and respective disclosures which are herein incorporated by reference in their entirety.

Alternatively, techniques described for the production of single chain antibodies (U.S. Patent 4,946,778; Bird, 1988, Science *242*:423-426; Huston *et al*., 1988, Proc. Natl. Acad. Sci. USA *85*:5879-5883; and Ward *et al*., 1989, Nature *341*:544-546) can be adapted to produce single chain antibodies against NHP sequence products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include, but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse *et al*., 1989, Science, *246*:1275-1281) to allow rapid and easy identification of monoclonal Fab fragments with the desired specificity.

Antibodies to a NHP can, in turn, be utilized to generate anti-idiotype antibodies that "mimic" a given NHP, using techniques well known to those skilled in the art. (See, e.g., Greenspan & Bona, 1993, FASEB J 7(*5*):437-444; and Missinoff, 1991, J. Immunol. *147*(*8*):2429-2438). For example antibodies which bind to a NHP domain and competitively inhibit the binding of NHP to its cognate receptor can be used to generate anti-idiotypes that "mimic" the NHP and, therefore, bind and activate or neutralize a receptor. Such anti-idiotypic antibodies or Fab fragments of such anti-idiotypes can be used in therapeutic regimens involving a NHP mediated pathway.

The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims. All cited publications, patents, and patent applications are herein incorporated by reference in their entirety.

## Claims

1. An isolated nucleic acid molecule comprising a nucleotide sequences described in SEQ ID NO:5, 7, 9, 11, 13, 15, 17, 19, 21 or 25.

2. An isolated nucleic acid molecule comprising a nucleic acid sequence that encodes the amino acid sequences described in SEQ ID NO:6, 8, 10, 12, 14, 16, 18, 20, 22 or 26.

3. An isolated polypeptide encoded by a nucleic acid as claimed in claim 1 or claim 2.

4. A host cell that comprises a nucleic acid molecule as claimed in claim 1 or claim 2.

5. An antibody that specifically recognises one or more epitopes or a polypeptide as claimed in claim3, or an antibody-binding fragment thereof.

6. A non-human transgenic animal that expresses a nucleic acid molecule as claimed in claim 1 or claim 2.

7. Use of a nucleic acid molecule as claimed in claim 1 or claim 2, a polypeptides as claimed in claim 3, a host cell as claimed in claim 4, an antibody or epitope-binding fragment thereof as claimed in claim 5, or a non-human transgenic animal as claimed in claim 6 in diagnosis, drug screening, clinical trial monitoring, the treatment of diseases and disorders, or cosmetic or nutriceutical applications.


SEQUENCE LISTING

<110> LEXICON GENETICS INCORPORATED

<120> NOVEL HUMAN MEMBRANE PROTEINS AND
  POLYNUCLEOTIDES ENCODING THE SAME

<130> 8568(1)EP/TJH

<150> US 60/178,557
<151> 2000-01-26

<160> 27

<170> FastSEQ for Windows Version 4.0

<210> 1
<211> 3924
<212> DNA
<213> homo sapiens

<400> 1
atggattctt taccacggct gaccagcgtt ttgactttgc tgttctctgg cttgtggcat 60
ttaggattaa cagcgacaaa ctacaactgt gatgatccac tagcatccct gctctctcca 120
atggcttttt ccagttcctc agacctcact ggcactcaca gcccagctca actcaactgg 180
agagttggaa ctggcggttg gtccccagca gattccaatg ctcaacagtg gctccagatg 240
gacctgggaa acagagtaga gattacagca gtggccacgc agggaagata cggaagctct 300
gactgggtga cgagttacag cctgatgttc agtgacacag gacgcaactg gaaacagtac 360
aaacaagaag acagcatctg gacctttgca ggaaacatga atgctgacag cgtggtgcac 420
cacaagctat tgcactcagt gagagcccga tttgttcgct ttgtgcccct ggaatggaat 480
cccagtggga agattggcat gagagtcgag gtctacggat gttcctataa atcagacgtt 540
gctgactttg atggccgaag ctcacttctg tacaggttca atcagaagtt gatgagtact 600
ctcaaagatg tgatctccct gaagttcaag agcatgcaag gagatggggt cctgttccat 660
ggagaaggtc agcgtggaga ccacatcacc ttggaactcc agaaggggag gctcgcccta 720
cacctcaatt tgggtgacag caaagcgcgg ctcagcagca gcttgccctc tgccaccctg 780
ggcagcctcc tggatgacca gcactggcac tyggtcctca ttgagcgggt gggcaagcag 840
gtgaacttca cggtggacaa gcacacacag cacttccgca ccaagggcga gacggatgcc 900
ttagacattg actatgagct tagttttgga ggaattccag taccaggaaa acctgggacc 960
tttttaaaga aaaacttcca tggatgcatc gaaaaccttt actacaatgg agtaaacata 1020
attracctgg ctaagagacg aaagcatcag atctatactg tgggcaatgt cactttttcc 1080
tgctccgaac cacagattgt gcccatcaca tttgtyaact ccagcggcag ctatttgctg 1140
ctgcccggca ccccccaaat tgatgggctc tcagtgagtt tccagtttcg aacatggaac 1200
aaggatggtc tgcttctgtc cacagagctg tctgagggct cgggaaccct gctgctgagc 1260
ctggagggtg gaatcctgag actcgtgatt cagaaaatga cagaacgcgt agctgaaatc 1320
ctcacaggca gcaacttgaa tgatggcctg tggcactcgg ttagcatcaa cgccaggagg 1380
aaccgcatca cgctcactct ggatgatgaa gcagcacccc cggctccaga cagcacttgg 1440
gtgcagattt attctggaaa tagctactat tttggagggt gccccgacaa tctcaccgat 1500
tcccaatgtt taaatcccat taaggctttc caaggctgca tgaggctcat ctttattgat 1560
aaccagccca aggacctcat ttcagttcag caaggttccc tggggaattt tagtgattta 1620
cacattgatc tgtgtagcat caaagacagg tgtttgccaa actactgtga acatggagga 1680
agctgctccc agtcctggac taccttctat tgtaactgca gtgacacaag ttacactggt 1740
gccacctgcc acaactccat ctacgagcaa tcctgcgagg tgtacaggca ccaggggaat 1800
acagccggct tcttctacat cgactcagat ggcagcggcc cactgggacc tctccaggtg 1860
tactgcaata tcactgagga caagatctgg acatcagtgc agcacaacaa tacagagctg 1920
acccgagtgc ggggcgctaa ccctgagaag ccctatgcca tggccttgga ctacgggggc 1980
agcatggaac agctggaggc cgtgatcgac ggctctgagc actgtgagca ggaggtggcc 2040
taccactgca ggaggtcccg cctgctcaac acgccggatg gaacaccatt tacctggtgg 2100
attgggcggt ccaatgaaag gcacccttac tggggaggtt cccctcctgg ggtccagcag 2160
tgtgagtgtg gcctagacga gagctgcctg gacattcagc acttttgcaa ttgcgacgct 2220
gacaaggatg aatggacaaa tgatactggc tttctttcct tcaaagacca cttgcctgtc 2280
actcagatag ttatcactga taccgacaga tcaaactcag aagccgcttg gagaattggt 2340
cccttgcgtt gctatggtga ccgacgcttc tggaacgccg tctcatttta tacagaagcc 2400
tcttacctcc actttcctac cttccatgcg gaattcagtg ccgatatttc cttctttttt 2460
aaaaccacag cattatccgg agttttccta gaaaatcttg gcattaaaga cttcattcga 2520
ctcgaaataa gctctccttc agagatcacc tttgccatcg atgttgggaa tggtcctgtg 2580
gagcttgtag tccagtctcc ttctcttctg aatgacaacc aatggcacta tgtccgggct 2640

gagaggaacc tcaaggagac ctccctgcag gtggacaacc ttccaaggag caccagggag 2700
acgtcggagg agggccattt tcgactgcag ctgaacagcc agttgtttgt agggggaacg 2760
tcatccagac agaaaggctt cctaggatgc attcgctcct tacacttgaa tggacagaaa 2820
atggacctgg aagagagggc aaaggtcaca tctggagtca ggccaggctg ccccggccac 2880
tgcagcagct acggcagcat ctgccacaac gggggcaagt gtgtggagaa gcacaatggc 2940
tacctgtgtg attgcaccaa ttcaccttat gaagggccct tttgcaaaaa agaggtttct 3000
gctgtttttg aggctggcac gtcggttact tacatgtttc aagaacccta tcctgtgacc 3060
aagaatataa gcctctcatc ctcagctatt tacacagatt cagctccatc caaggaaaac 3120
attgcactta gctttgtgac aacccaggca cccagtcttt tgctctttat caattcttct 3180
tctcaggact tcgtggttgt tctgctctgc aagaatggaa gcttacaggt tcgctatcac 3240
ctaaacaagg aagaaaccca tgtattcacc attgatgcag ataactttgc taacagaagg 3300
atgcaccact tgaagattaa ccgagaggga agagagctta ccattcagat ggaccagcaa 3360
cttcgactca gttataactt ctctccggaa gtagagttca gggttataag gtcactcacc 3420
ttgggcaaag tcacagagaa tcttggtttg gattctgaag ttgctaaagc aaatgccatg 3480
ggttttgctg gatgcatgtc ttccgtccag tacaaccaca tagcaccact gaaggctgcc 3540
ctgcgccatg ccactgtcgc gcctgtgact gtccatggga ccttgacgga atccagctgt 3600
ggcttcatgg tggactcaga tgtgaatgca gtgaccacgg tgcattcttc atcagatcct 3660
tttgggaaga cagatgagcg ggaaccactc acaaatgctg ttcgaagtga ttcggcagtc 3720
atcggagggg tgatagcagt ggtgatattc atcatcttct gtatcatcgg catcatgacc 3780
cggttcctct accagcacaa gcagtcacat cgtacgagcc agatgaagga gaaggaatat 3840
ccagaaaatt tggacagttc cttcagaaat gaaattgact tgcaaaacac agtgagcgag 3900
tgtaaacggg aatatttcat ctga 3924

<210> 2
<211> 1307
<212> PRT
<213> homo sapiens

<220>
<221> VARIANT
<222> 271, 342
<223> Xaa = Any Amino Acid

<400> 2
Met Asp Ser Leu Pro Arg Leu Thr Ser Val Leu Thr Leu Leu Phe Ser
1               5                   10                  15
Gly Leu Trp His Leu Gly Leu Thr Ala Thr Asn Tyr Asn Cys Asp Asp
            20                  25                  30
Pro Leu Ala Ser Leu Leu Ser Pro Met Ala Phe Ser Ser Ser Ser Asp
        35                  40                  45
Leu Thr Gly Thr His Ser Pro Ala Gln Leu Asn Trp Arg Val Gly Thr
    50                  55                  60
Gly Gly Trp Ser Pro Ala Asp Ser Asn Ala Gln Gln Trp Leu Gln Met
65                  70                  75                  80
Asp Leu Gly Asn Arg Val Glu Ile Thr Ala Val Ala Thr Gln Gly Arg
                85                  90                  95
Tyr Gly Ser Ser Asp Trp Val Thr Ser Tyr Ser Leu Met Phe Ser Asp
            100                 105                 110
Thr Gly Arg Asn Trp Lys Gln Tyr Lys Gln Glu Asp Ser Ile Trp Thr
        115                 120                 125
Phe Ala Gly Asn Met Asn Ala Asp Ser Val Val His His Lys Leu Leu
    130                 135                 140
His Ser Val Arg Ala Arg Phe Val Arg Phe Val Pro Leu Glu Trp Asn
145                 150                 155                 160
Pro Ser Gly Lys Ile Gly Met Arg Val Glu Val Tyr Gly Cys Ser Tyr
                165                 170                 175
Lys Ser Asp Val Ala Asp Phe Asp Gly Arg Ser Ser Leu Leu Tyr Arg
            180                 185                 190
Phe Asn Gln Lys Leu Met Ser Thr Leu Lys Asp Val Ile Ser Leu Lys
        195                 200                 205
Phe Lys Ser Met Gln Gly Asp Gly Val Leu Phe His Gly Glu Gly Gln
    210                 215                 220
Arg Gly Asp His Ile Thr Leu Glu Leu Gln Lys Gly Arg Leu Ala Leu
225                 230                 235                 240
His Leu Asn Leu Gly Asp Ser Lys Ala Arg Leu Ser Ser Ser Leu Pro
                245                 250                 255
Ser Ala Thr Leu Gly Ser Leu Leu Asp Asp Gln His Trp His Xaa Val
            260                 265                 270

Leu Ile Glu Arg Val Gly Lys Gln Val Asn Phe Thr Val Asp Lys His
        275                 280                 285
Thr Gln His Phe Arg Thr Lys Gly Glu Thr Asp Ala Leu Asp Ile Asp
    290                 295                 300
Tyr Glu Leu Ser Phe Gly Gly Ile Pro Val Pro Gly Lys Pro Gly Thr
305                 310                 315                 320
Phe Leu Lys Lys Asn Phe His Gly Cys Ile Glu Asn Leu Tyr Tyr Asn
                325                 330                 335
Gly Val Asn Ile Ile Xaa Leu Ala Lys Arg Arg Lys His Gln Ile Tyr
            340                 345                 350
Thr Val Gly Asn Val Thr Phe Ser Cys Ser Glu Pro Gln Ile Val Pro
        355                 360                 365
Ile Thr Phe Val Asn Ser Ser Gly Ser Tyr Leu Leu Leu Pro Gly Thr
    370                 375                 380
Pro Gln Ile Asp Gly Leu Ser Val Ser Phe Gln Phe Arg Thr Trp Asn
385                 390                 395                 400
Lys Asp Gly Leu Leu Leu Ser Thr Glu Leu Ser Glu Gly Ser Gly Thr
                405                 410                 415
Leu Leu Leu Ser Leu Glu Gly Gly Ile Leu Arg Leu Val Ile Gln Lys
            420                 425                 430
Met Thr Glu Arg Val Ala Glu Ile Leu Thr Gly Ser Asn Leu Asn Asp
        435                 440                 445
Gly Leu Trp His Ser Val Ser Ile Asn Ala Arg Arg Asn Arg Ile Thr
    450                 455                 460
Leu Thr Leu Asp Asp Glu Ala Ala Pro Pro Ala Pro Asp Ser Thr Trp
465                 470                 475                 480
Val Gln Ile Tyr Ser Gly Asn Ser Tyr Tyr Phe Gly Gly Cys Pro Asp
                485                 490                 495
Asn Leu Thr Asp Ser Gln Cys Leu Asn Pro Ile Lys Ala Phe Gln Gly
            500                 505                 510
Cys Met Arg Leu Ile Phe Ile Asp Asn Gln Pro Lys Asp Leu Ile Ser
        515                 520                 525
Val Gln Gln Gly Ser Leu Gly Asn Phe Ser Asp Leu His Ile Asp Leu
    530                 535                 540
Cys Ser Ile Lys Asp Arg Cys Leu Pro Asn Tyr Cys Glu His Gly Gly
545                 550                 555                 560
Ser Cys Ser Gln Ser Trp Thr Thr Phe Tyr Cys Asn Cys Ser Asp Thr
                565                 570                 575
Ser Tyr Thr Gly Ala Thr Cys His Asn Ser Ile Tyr Glu Gln Ser Cys
            580                 585                 590
Glu Val Tyr Arg His Gln Gly Asn Thr Ala Gly Phe Phe Tyr Ile Asp
        595                 600                 605
Ser Asp Gly Ser Gly Pro Leu Gly Pro Leu Gln Val Tyr Cys Asn Ile
    610                 615                 620
Thr Glu Asp Lys Ile Trp Thr Ser Val Gln His Asn Asn Thr Glu Leu
625                 630                 635                 640
Thr Arg Val Arg Gly Ala Asn Pro Glu Lys Pro Tyr Ala Met Ala Leu
                645                 650                 655
Asp Tyr Gly Gly Ser Met Glu Gln Leu Glu Ala Val Ile Asp Gly Ser
            660                 665                 670
Glu His Cys Glu Gln Glu Val Ala Tyr His Cys Arg Arg Ser Arg Leu
        675                 680                 685
Leu Asn Thr Pro Asp Gly Thr Pro Phe Thr Trp Trp Ile Gly Arg Ser
    690                 695                 700
Asn Glu Arg His Pro Tyr Trp Gly Gly Ser Pro Pro Gly Val Gln Gln
705                 710                 715                 720
Cys Glu Cys Gly Leu Asp Glu Ser Cys Leu Asp Ile Gln His Phe Cys
                725                 730                 735
Asn Cys Asp Ala Asp Lys Asp Glu Trp Thr Asn Asp Thr Gly Phe Leu
            740                 745                 750
Ser Phe Lys Asp His Leu Pro Val Thr Gln Ile Val Ile Thr Asp Thr
        755                 760                 765
Asp Arg Ser Asn Ser Glu Ala Ala Trp Arg Ile Gly Pro Leu Arg Cys
    770                 775                 780
Tyr Gly Asp Arg Arg Phe Trp Asn Ala Val Ser Phe Tyr Thr Glu Ala
785                 790                 795                 800
Ser Tyr Leu His Phe Pro Thr Phe His Ala Glu Phe Ser Ala Asp Ile
                805                 810                 815

Ser Phe Phe Phe Lys Thr Thr Ala Leu Ser Gly Val Phe Leu Glu Asn
            820                 825                 830
Leu Gly Ile Lys Asp Phe Ile Arg Leu Glu Ile Ser Ser Pro Ser Glu
        835                 840                 845
Ile Thr Phe Ala Ile Asp Val Gly Asn Gly Pro Val Glu Leu Val Val
    850                 855                 860
Gln Ser Pro Ser Leu Leu Asn Asp Asn Gln Trp His Tyr Val Arg Ala
865                 870                 875                 880
Glu Arg Asn Leu Lys Glu Thr Ser Leu Gln Val Asp Asn Leu Pro Arg
                885                 890                 895
Ser Thr Arg Glu Thr Ser Glu Glu Gly His Phe Arg Leu Gln Leu Asn
            900                 905                 910
Ser Gln Leu Phe Val Gly Gly Thr Ser Ser Arg Gln Lys Gly Phe Leu
        915                 920                 925
Gly Cys Ile Arg Ser Leu His Leu Asn Gly Gln Lys Met Asp Leu Glu
    930                 935                 940
Glu Arg Ala Lys Val Thr Ser Gly Val Arg Pro Gly Cys Pro Gly His
945                 950                 955                 960
Cys Ser Ser Tyr Gly Ser Ile Cys His Asn Gly Gly Lys Cys Val Glu
                965                 970                 975
Lys His Asn Gly Tyr Leu Cys Asp Cys Thr Asn Ser Pro Tyr Glu Gly
            980                 985                 990
Pro Phe Cys Lys Lys Glu Val Ser Ala Val Phe Glu Ala Gly Thr Ser
        995                 1000                1005
Val Thr Tyr Met Phe Gln Glu Pro Tyr Pro Val Thr Lys Asn Ile Ser
    1010                1015                1020
Leu Ser Ser Ser Ala Ile Tyr Thr Asp Ser Ala Pro Ser Lys Glu Asn
1025                1030                1035                1040
Ile Ala Leu Ser Phe Val Thr Thr Gln Ala Pro Ser Leu Leu Leu Phe
                1045                1050                1055
Ile Asn Ser Ser Ser Gln Asp Phe Val Val Val Leu Leu Cys Lys Asn
            1060                1065                1070
Gly Ser Leu Gln Val Arg Tyr His Leu Asn Lys Glu Glu Thr His Val
        1075                1080                1085
Phe Thr Ile Asp Ala Asp Asn Phe Ala Asn Arg Arg Met His His Leu
    1090                1095                1100
Lys Ile Asn Arg Glu Gly Arg Glu Leu Thr Ile Gln Met Asp Gln Gln
1105                1110                1115                1120
Leu Arg Leu Ser Tyr Asn Phe Ser Pro Glu Val Glu Phe Arg Val Ile
                1125                1130                1135
Arg Ser Leu Thr Leu Gly Lys Val Thr Glu Asn Leu Gly Leu Asp Ser
            1140                1145                1150
Glu Val Ala Lys Ala Asn Ala Met Gly Phe Ala Gly Cys Met Ser Ser
        1155                1160                1165
Val Gln Tyr Asn His Ile Ala Pro Leu Lys Ala Ala Leu Arg His Ala
    1170                1175                1180
Thr Val Ala Pro Val Thr Val His Gly Thr Leu Thr Glu Ser Ser Cys
1185                1190                1195                1200
Gly Phe Met Val Asp Ser Asp Val Asn Ala Val Thr Thr Val His Ser
                1205                1210                1215
Ser Ser Asp Pro Phe Gly Lys Thr Asp Glu Arg Glu Pro Leu Thr Asn
            1220                1225                1230
Ala Val Arg Ser Asp Ser Ala Val Ile Gly Gly Val Ile Ala Val Val
        1235                1240                1245
Ile Phe Ile Ile Phe Cys Ile Ile Gly Ile Met Thr Arg Phe Leu Tyr
    1250                1255                1260
Gln His Lys Gln Ser His Arg Thr Ser Gln Met Lys Glu Lys Glu Tyr
1265                1270                1275                1280
Pro Glu Asn Leu Asp Ser Ser Phe Arg Asn Glu Ile Asp Leu Gln Asn
                1285                1290                1295
Thr Val Ser Glu Cys Lys Arg Glu Tyr Phe Ile
            1300                1305

<210> 3
<211> 3780
<212> DNA
<213> homo sapiens

<400> 3
atggattctt taccacggct gaccagcgtt ttgactttgc tgttctctgg cttgtggcat 60
ttaggattaa cagcgacaaa ctacaactgt gatgatccac tagcatccct gctctctcca 120
atggcttttt ccagttcctc agacctcact ggcactcaca gcccagctca actcaactgg 180
agagttggaa ctggcggttg gtccccagca gattccaatg ctcaacagtg gctccagatg 240
gacctgggaa acagagtaga gattacagca gtggccacgc agggaagata cggaagctct 300
gactgggtga cgagttacag cctgatgttc agtgacacag gacgcaactg gaaacagtac 360
aaacaagaag acagcatctg gacctttgca ggaaacatga atgctgacag cgtggtgcac 420
cacaagctat tgcactcagt gagagcccga tttgttcgct ttgtgcccct ggaatggaat 480
cccagtggga agattggcat gagagtcgag gtctacggat gttcctataa atcagacgtt 540
gctgactttg atggccgaag ctcacttctg tacaggttca atcagaagtt gatgagtact 600
ctcaaagatg tgatctccct gaagttcaag agcatgcaag gagatggggt cctgttccat 660
ggagaaggtc agcgtggaga ccacatcacc ttggaactcc agaaggggag gctcgcccta 720
cacctcaatt tgggtgacag caaagcgcgg ctcagcagca gcttgccctc tgccaccctg 780
ggcagcctcc tggatgacca gcactggcac tyggtcctca ttgagcgggt gggcaagcag 840
gtgaacttca cggtggacaa gcacacacag cacttccgca ccaagggcga gacggatgcc 900
ttagacattg actatgaggg caatgtcact ttttcctgct ccgaaccaca gattgtgccc 960
atcacatttg tyaactccag cggcagctat ttgctgctgc ccggcacccc ccaaattgat 1020
gggctctcag tgagtttcca gtttcgaaca tggaacaagg atggtctgct tctgtccaca 1080
gagctgtctg agggctcggg aaccctgctg ctgagcctgg agggtggaat cctgagactc 1140
gtgattcaga aaatgacaga acgcgtagct gaaatcctca caggcagcaa cttgaatgat 1200
ggcctgtggc actcggttag catcaacgcc aggaggaacc gcatcacgct cactctggat 1260
gatgaagcag cacccccggc tccagacagc acttgggtgc agatttattc tggaaatagc 1320
tactattttg gagggtgccc cgacaatctc accgattccc aatgtttaaa tcccattaag 1380
gctttccaag gctgcatgag gctcatcttt attgataacc agcccaagga cctcatttca 1440
gttcagcaag gttccctggg gaattttagt gatttacaca ttgatctgtg tagcatcaaa 1500
gacaggtgtt tgccaaacta ctgtgaacat ggaggaagct gctcccagtc ctggactacc 1560
ttctattgta actgcagtga cacaagttac actggtgcca cctgccacaa ctccatctac 1620
gagcaatcct gcgaggtgta caggcaccag gggaatacag ccggcttctt ctacatcgac 1680
tcagatggca gcggcccact gggacctctc caggtgtact gcaatatcac tgaggacaag 1740
atctggacat cagtgcagca caacaataca gagctgaccc gagtgcgggg cgctaaccct 1800
gagaagccct atgccatggc cttggactac gggggcagca tggaacagct ggaggccgtg 1860
atcgacggct ctgagcactg tgagcaggag gtggcctacc actgcaggag gtcccgcctg 1920
ctcaacacgc cggatggaac accatttacc tggtggattg ggcggtccaa tgaaaggcac 1980
ccttactggg gaggttcccc tcctggggtc cagcagtgtg agtgtggcct agacgagagc 2040
tgcctggaca ttcagcactt ttgcaattgc gacgctgaca aggatgaatg gacaaatgat 2100
actggctttc tttccttcaa agaccacttg cctgtcactc agatagttat cactgatacc 2160
gacagatcaa actcagaagc cgcttggaga attggtccct tgcgttgcta tggtgaccga 2220
cgcttctgga acgccgtctc attttataca gaagcctctt acctccactt tcctaccttc 2280
catgcggaat tcagtgccga tatttccttc tttttaaaa ccacagcatt atccggagtt 2340
ttcctagaaa atcttggcat taaagacttc attcgactcg aaataagctc tccttcagag 2400
atcacctttg ccatcgatgt tgggaatggt cctgtggagc ttgtagtcca gtctccttct 2460
cttctgaatg acaaccaatg gcactatgtc cgggctgaga ggaacctcaa ggagacctcc 2520
ctgcaggtgg acaaccttcc aaggagcacc agggagacgt cggaggaggg ccatttcga 2580
ctgcagctga acagccagtt gtttgtaggg ggaacgtcat ccagacagaa aggcttccta 2640
ggatgcattc gctccttaca cttgaatgga cagaaaatgg acctggaaga gagggcaaag 2700
gtcacatctg gagtcaggcc aggctgcccc ggccactgca gcagctacgg cagcatctgc 2760
cacaacgggg gcaagtgtgt ggagaagcac aatggctacc tgtgtgattg caccaattca 2820
ccttatgaag ggcccttttg caaaaaagag gtttctgctg tttttgaggc tggcacgtcg 2880
gttacttaca tgtttcaaga accctatcct gtgaccaaga atataagcct ctcatcctca 2940
gctatttaca cagattcagc tccatccaag gaaaacattg cacttagctt tgtgacaacc 3000
caggcaccca gtcttttgct ctttatcaat tcttcttctc aggacttcgt ggttgttctg 3060
ctctgcaaga atggaagctt acaggttcgc tatcacctaa acaaggaaga aacccatgta 3120
ttcaccattg atgcagataa ctttgctaac agaaggatgc accacttgaa gattaaccga 3180
gagggaagag agcttaccat tcagatggac cagcaacttc gactcagtta taacttctct 3240
ccggaagtag agttcagggt tataaggtca ctcaccttgg gcaaagtcac agagaatctt 3300
ggtttggatt ctgaagttgc taaagcaaat gccatgggtt ttgctggatg catgtcttcc 3360
gtccagtaca accacatagc accactgaag gctgccctgc gccatgccac tgtcgcgcct 3420
gtgactgtcc atgggacctt gacggaatcc agctgtggct tcatggtgga ctcagatgtg 3480
aatgcagtga ccacggtgca ttcttcatca gatccttttg ggaagacaga tgagcgggaa 3540
ccactcacaa atgctgttcg aagtgattcg gcagtcatcg gaggggtgat agcagtggtg 3600
atattcatca tcttctgtat catcggcatc atgacccggt tcctctacca gcacaagcag 3660
tcacatcgta cgagccagat gaaggagaag gaatatccag aaaatttgga cagttccttc 3720
agaaatgaaa ttgacttgca aaacacagtg agcgagtgta aacgggaata tttcatctga 3780

<210> 4

<211> 1259
<212> PRT
<213> homo sapiens

<220>
<221> VARIANT
<222> 271
<223> Xaa = Any Amino Acid

<400> 4
Met Asp Ser Leu Pro Arg Leu Thr Ser Val Leu Thr Leu Leu Phe Ser
1               5                   10                  15
Gly Leu Trp His Leu Gly Leu Thr Ala Thr Asn Tyr Asn Cys Asp Asp
            20                  25                  30
Pro Leu Ala Ser Leu Leu Ser Pro Met Ala Phe Ser Ser Ser Ser Asp
        35                  40                  45
Leu Thr Gly Thr His Ser Pro Ala Gln Leu Asn Trp Arg Val Gly Thr
    50                  55                  60
Gly Gly Trp Ser Pro Ala Asp Ser Asn Ala Gln Gln Trp Leu Gln Met
65                  70                  75                  80
Asp Leu Gly Asn Arg Val Glu Ile Thr Ala Val Ala Thr Gln Gly Arg
                85                  90                  95
Tyr Gly Ser Ser Asp Trp Val Thr Ser Tyr Ser Leu Met Phe Ser Asp
            100                 105                 110
Thr Gly Arg Asn Trp Lys Gln Tyr Lys Gln Glu Asp Ser Ile Trp Thr
        115                 120                 125
Phe Ala Gly Asn Met Asn Ala Asp Ser Val Val His His Lys Leu Leu
    130                 135                 140
His Ser Val Arg Ala Arg Phe Val Arg Phe Val Pro Leu Glu Trp Asn
145                 150                 155                 160
Pro Ser Gly Lys Ile Gly Met Arg Val Glu Val Tyr Gly Cys Ser Tyr
                165                 170                 175
Lys Ser Asp Val Ala Asp Phe Asp Gly Arg Ser Ser Leu Leu Tyr Arg
            180                 185                 190
Phe Asn Gln Lys Leu Met Ser Thr Leu Lys Asp Val Ile Ser Leu Lys
        195                 200                 205
Phe Lys Ser Met Gln Gly Asp Gly Val Leu Phe His Gly Glu Gly Gln
    210                 215                 220
Arg Gly Asp His Ile Thr Leu Glu Leu Gln Lys Gly Arg Leu Ala Leu
225                 230                 235                 240
His Leu Asn Leu Gly Asp Ser Lys Ala Arg Leu Ser Ser Ser Leu Pro
                245                 250                 255
Ser Ala Thr Leu Gly Ser Leu Leu Asp Asp Gln His Trp His Xaa Val
            260                 265                 270
Leu Ile Glu Arg Val Gly Lys Gln Val Asn Phe Thr Val Asp Lys His
        275                 280                 285
Thr Gln His Phe Arg Thr Lys Gly Glu Thr Asp Ala Leu Asp Ile Asp
    290                 295                 300
Tyr Glu Gly Asn Val Thr Phe Ser Cys Ser Glu Pro Gln Ile Val Pro
305                 310                 315                 320
Ile Thr Phe Val Asn Ser Ser Gly Ser Tyr Leu Leu Leu Pro Gly Thr
                325                 330                 335
Pro Gln Ile Asp Gly Leu Ser Val Ser Phe Gln Phe Arg Thr Trp Asn
            340                 345                 350
Lys Asp Gly Leu Leu Leu Ser Thr Glu Leu Ser Glu Gly Ser Gly Thr
        355                 360                 365
Leu Leu Leu Ser Leu Glu Gly Gly Ile Leu Arg Leu Val Ile Gln Lys
    370                 375                 380
Met Thr Glu Arg Val Ala Glu Ile Leu Thr Gly Ser Asn Leu Asn Asp
385                 390                 395                 400
Gly Leu Trp His Ser Val Ser Ile Asn Ala Arg Arg Asn Arg Ile Thr
                405                 410                 415
Leu Thr Leu Asp Asp Glu Ala Ala Pro Pro Ala Pro Asp Ser Thr Trp
            420                 425                 430
Val Gln Ile Tyr Ser Gly Asn Ser Tyr Tyr Phe Gly Gly Cys Pro Asp
        435                 440                 445
Asn Leu Thr Asp Ser Gln Cys Leu Asn Pro Ile Lys Ala Phe Gln Gly
    450                 455                 460

Cys Met Arg Leu Ile Phe Ile Asp Asn Gln Pro Lys Asp Leu Ile Ser
465                 470                 475                 480
Val Gln Gln Gly Ser Leu Gly Asn Phe Ser Asp Leu His Ile Asp Leu
                485                 490                 495
Cys Ser Ile Lys Asp Arg Cys Leu Pro Asn Tyr Cys Glu His Gly Gly
            500                 505                 510
Ser Cys Ser Gln Ser Trp Thr Thr Phe Tyr Cys Asn Cys Ser Asp Thr
        515                 520                 525
Ser Tyr Thr Gly Ala Thr Cys His Asn Ser Ile Tyr Glu Gln Ser Cys
    530                 535                 540
Glu Val Tyr Arg His Gln Gly Asn Thr Ala Gly Phe Phe Tyr Ile Asp
545                 550                 555                 560
Ser Asp Gly Ser Gly Pro Leu Gly Pro Leu Gln Val Tyr Cys Asn Ile
                565                 570                 575
Thr Glu Asp Lys Ile Trp Thr Ser Val Gln His Asn Asn Thr Glu Leu
            580                 585                 590
Thr Arg Val Arg Gly Ala Asn Pro Glu Lys Pro Tyr Ala Met Ala Leu
        595                 600                 605
Asp Tyr Gly Gly Ser Met Glu Gln Leu Glu Ala Val Ile Asp Gly Ser
    610                 615                 620
Glu His Cys Glu Gln Glu Val Ala Tyr His Cys Arg Arg Ser Arg Leu
625                 630                 635                 640
Leu Asn Thr Pro Asp Gly Thr Pro Phe Thr Trp Trp Ile Gly Arg Ser
                645                 650                 655
Asn Glu Arg His Pro Tyr Trp Gly Gly Ser Pro Pro Gly Val Gln Gln
            660                 665                 670
Cys Glu Cys Gly Leu Asp Glu Ser Cys Leu Asp Ile Gln His Phe Cys
        675                 680                 685
Asn Cys Asp Ala Asp Lys Asp Glu Trp Thr Asn Asp Thr Gly Phe Leu
    690                 695                 700
Ser Phe Lys Asp His Leu Pro Val Thr Gln Ile Val Ile Thr Asp Thr
705                 710                 715                 720
Asp Arg Ser Asn Ser Glu Ala Ala Trp Arg Ile Gly Pro Leu Arg Cys
                725                 730                 735
Tyr Gly Asp Arg Arg Phe Trp Asn Ala Val Ser Phe Tyr Thr Glu Ala
            740                 745                 750
Ser Tyr Leu His Phe Pro Thr Phe His Ala Glu Phe Ser Ala Asp Ile
        755                 760                 765
Ser Phe Phe Phe Lys Thr Thr Ala Leu Ser Gly Val Phe Leu Glu Asn
    770                 775                 780
Leu Gly Ile Lys Asp Phe Ile Arg Leu Glu Ile Ser Ser Pro Ser Glu
785                 790                 795                 800
Ile Thr Phe Ala Ile Asp Val Gly Asn Gly Pro Val Glu Leu Val Val
                805                 810                 815
Gln Ser Pro Ser Leu Leu Asn Asp Asn Gln Trp His Tyr Val Arg Ala
            820                 825                 830
Glu Arg Asn Leu Lys Glu Thr Ser Leu Gln Val Asp Asn Leu Pro Arg
        835                 840                 845
Ser Thr Arg Glu Thr Ser Glu Glu Gly His Phe Arg Leu Gln Leu Asn
    850                 855                 860
Ser Gln Leu Phe Val Gly Gly Thr Ser Ser Arg Gln Lys Gly Phe Leu
865                 870                 875                 880
Gly Cys Ile Arg Ser Leu His Leu Asn Gly Gln Lys Met Asp Leu Glu
                885                 890                 895
Glu Arg Ala Lys Val Thr Ser Gly Val Arg Pro Gly Cys Pro Gly His
            900                 905                 910
Cys Ser Ser Tyr Gly Ser Ile Cys His Asn Gly Gly Lys Cys Val Glu
        915                 920                 925
Lys His Asn Gly Tyr Leu Cys Asp Cys Thr Asn Ser Pro Tyr Glu Gly
    930                 935                 940
Pro Phe Cys Lys Lys Glu Val Ser Ala Val Phe Glu Ala Gly Thr Ser
945                 950                 955                 960
Val Thr Tyr Met Phe Gln Glu Pro Tyr Pro Val Thr Lys Asn Ile Ser
                965                 970                 975
Leu Ser Ser Ser Ala Ile Tyr Thr Asp Ser Ala Pro Ser Lys Glu Asn
            980                 985                 990
Ile Ala Leu Ser Phe Val Thr Thr Gln Ala Pro Ser Leu Leu Leu Phe
        995                 1000                1005

Ile Asn Ser Ser Ser Gln Asp Phe Val Val Val Leu Leu Cys Lys Asn
    1010                1015                1020
Gly Ser Leu Gln Val Arg Tyr His Leu Asn Lys Glu Glu Thr His Val
1025                1030                1035                1040
Phe Thr Ile Asp Ala Asp Asn Phe Ala Asn Arg Arg Met His His Leu
                1045                1050                1055
Lys Ile Asn Arg Glu Gly Arg Glu Leu Thr Ile Gln Met Asp Gln Gln
            1060                1065                1070
Leu Arg Leu Ser Tyr Asn Phe Ser Pro Glu Val Glu Phe Arg Val Ile
        1075                1080                1085
Arg Ser Leu Thr Leu Gly Lys Val Thr Glu Asn Leu Gly Leu Asp Ser
    1090                1095                1100
Glu Val Ala Lys Ala Asn Ala Met Gly Phe Ala Gly Cys Met Ser Ser
1105                1110                1115                1120
Val Gln Tyr Asn His Ile Ala Pro Leu Lys Ala Ala Leu Arg His Ala
                1125                1130                1135
Thr Val Ala Pro Val Thr Val His Gly Thr Leu Thr Glu Ser Ser Cys
            1140                1145                1150
Gly Phe Met Val Asp Ser Asp Val Asn Ala Val Thr Thr Val His Ser
        1155                1160                1165
Ser Ser Asp Pro Phe Gly Lys Thr Asp Glu Arg Glu Pro Leu Thr Asn
    1170                1175                1180
Ala Val Arg Ser Asp Ser Ala Val Ile Gly Gly Val Ile Ala Val Val
1185                1190                1195                1200
Ile Phe Ile Ile Phe Cys Ile Ile Gly Ile Met Thr Arg Phe Leu Tyr
                1205                1210                1215
Gln His Lys Gln Ser His Arg Thr Ser Gln Met Lys Glu Lys Glu Tyr
            1220                1225                1230
Pro Glu Asn Leu Asp Ser Ser Phe Arg Asn Glu Ile Asp Leu Gln Asn
        1235                1240                1245
Thr Val Ser Glu Cys Lys Arg Glu Tyr Phe Ile
    1250                1255

<210> 5
<211> 108
<212> DNA
<213> homo sapiens

<400> 5
atggattctt taccacggct gaccagcgtt ttgactttgc tgttctctgg cttgtggcat 60
ttaggattaa cagcgacaaa ctacctttgc aggaaacatg aatgctga                108

<210> 6
<211> 35
<212> PRT
<213> homo sapiens

<400> 6
Met Asp Ser Leu Pro Arg Leu Thr Ser Val Leu Thr Leu Leu Phe Ser
1               5                   10                  15
Gly Leu Trp His Leu Gly Leu Thr Ala Thr Asn Tyr Leu Cys Arg Lys
            20                  25                  30
His Glu Cys
        35

<210> 7
<211> 753
<212> DNA
<213> homo sapiens

<400> 7
atggattctt taccacggct gaccagcgtt ttgactttgc tgttctctgg cttgtggcat 60
ttaggattaa cagcgacaaa ctacaactgt gatgatccac tagcatccct gctctctcca 120
atggcttttt ccagttcctc agacctcact ggcactcaca gcccagctca actcaactgg 180
agagttggaa ctggcggttg gtccccagca gattccaatg ctcaacagtg gctccagatg 240
gacctgggaa acagagtaga gattacagca gtggccacgc agggaagata cggaagctct 300

gactgggtga cgagttacag cctgatgttc agtgacacag gacgcaactg gaaacagtac 360
aaacaagaag acagcatctg gacctttgca ggaaacatga atgctgacag cgtggtgcac 420
cacaagctat tgcactcagt gagagcccga tttgttcgct ttgtgcccct ggaatggaat 480
cccagtggga agattggcat gagagtcgag gtctacggat gttcctataa atcagacgtt 540
gctgactttg atggccgaag ctcacttctg tacaggttca atcagaagtt gatgagtact 600
ctcaaagatg tgatctccct gaagttcaag agcatgcaag gagatggggt cctgttccat 660
ggagaaggtc agcgtggaga ccacatcacc ttggaactcc agaaggggag gctcgcccta 720
cacctcaatt tggtggtttg tagttctcct tga 753

<210> 8
<211> 250
<212> PRT
<213> homo sapiens

<400> 8
Met Asp Ser Leu Pro Arg Leu Thr Ser Val Leu Thr Leu Leu Phe Ser
1               5                   10                  15
Gly Leu Trp His Leu Gly Leu Thr Ala Thr Asn Tyr Asn Cys Asp Asp
            20                  25                  30
Pro Leu Ala Ser Leu Leu Ser Pro Met Ala Phe Ser Ser Ser Ser Asp
        35                  40                  45
Leu Thr Gly Thr His Ser Pro Ala Gln Leu Asn Trp Arg Val Gly Thr
    50                  55                  60
Gly Gly Trp Ser Pro Ala Asp Ser Asn Ala Gln Gln Trp Leu Gln Met
65                  70                  75                  80
Asp Leu Gly Asn Arg Val Glu Ile Thr Ala Val Ala Thr Gln Gly Arg
                85                  90                  95
Tyr Gly Ser Ser Asp Trp Val Thr Ser Tyr Ser Leu Met Phe Ser Asp
            100                 105                 110
Thr Gly Arg Asn Trp Lys Gln Tyr Lys Gln Glu Asp Ser Ile Trp Thr
        115                 120                 125
Phe Ala Gly Asn Met Asn Ala Asp Ser Val Val His His Lys Leu Leu
    130                 135                 140
His Ser Val Arg Ala Arg Phe Val Arg Phe Val Pro Leu Glu Trp Asn
145                 150                 155                 160
Pro Ser Gly Lys Ile Gly Met Arg Val Glu Val Tyr Gly Cys Ser Tyr
                165                 170                 175
Lys Ser Asp Val Ala Asp Phe Asp Gly Arg Ser Ser Leu Leu Tyr Arg
            180                 185                 190
Phe Asn Gln Lys Leu Met Ser Thr Leu Lys Asp Val Ile Ser Leu Lys
        195                 200                 205
Phe Lys Ser Met Gln Gly Asp Gly Val Leu Phe His Gly Glu Gly Gln
    210                 215                 220
Arg Gly Asp His Ile Thr Leu Glu Leu Gln Lys Gly Arg Leu Ala Leu
225                 230                 235                 240
His Leu Asn Leu Val Val Cys Ser Ser Pro
                245                 250

<210> 9
<211> 840
<212> DNA
<213> homo sapiens

<400> 9
atggattctt taccacggct gaccagcgtt ttgactttgc tgttctctgg cttgtggcat 60
ttaggattaa cagcgacaaa ctacaactgt gatgatccac tagcatccct gctctctcca 120
atggcttttt ccagttcctc agacctcact ggcactcaca gcccagctca actcaactgg 180
agagttggaa ctggcggttg gtccccagca gattccaatg ctcaacagtg gctccagatg 240
gacctgggaa acagagtaga gattacagca gtggccacgc agggaagata cggaagctct 300
gactgggtga cgagttacag cctgatgttc agtgacacag gacgcaactg gaaacagtac 360
aaacaagaag acagcatctg gacctttgca ggaaacatga atgctgacag cgtggtgcac 420
cacaagctat tgcactcagt gagagcccga tttgttcgct ttgtgcccct ggaatggaat 480
cccagtggga agattggcat gagagtcgag gtctacggat gttcctataa atcagacgtt 540
gctgactttg atggccgaag ctcacttctg tacaggttca atcagaagtt gatgagtact 600
ctcaaagatg tgatctccct gaagttcaag agcatgcaag gagatggggt cctgttccat 660
ggagaaggtc agcgtggaga ccacatcacc ttggaactcc agaaggggag gctcgcccta 720
cacctcaatt tgggtgacag caaagcgcgg ctaagcactt gccctctgcc accctgggca 780

gcctcctgga tgaccagcac tggcactygg tcctcattga gcgggtgggc aagcaggtga 840

<210> 10
<211> 279
<212> PRT
<213> homo sapiens

<220>
<221> VARIANT
<222> 270
<223> Xaa = Any Amino Acid

<400> 10
Met Asp Ser Leu Pro Arg Leu Thr Ser Val Leu Thr Leu Leu Phe Ser
1               5                   10                  15
Gly Leu Trp His Leu Gly Leu Thr Ala Thr Asn Tyr Asn Cys Asp Asp
            20                  25                  30
Pro Leu Ala Ser Leu Leu Ser Pro Met Ala Phe Ser Ser Ser Ser Asp
        35                  40                  45
Leu Thr Gly Thr His Ser Pro Ala Gln Leu Asn Trp Arg Val Gly Thr
    50                  55                  60
Gly Gly Trp Ser Pro Ala Asp Ser Asn Ala Gln Gln Trp Leu Gln Met
65                  70                  75                  80
Asp Leu Gly Asn Arg Val Glu Ile Thr Ala Val Ala Thr Gln Gly Arg
                85                  90                  95
Tyr Gly Ser Ser Asp Trp Val Thr Ser Tyr Ser Leu Met Phe Ser Asp
            100                 105                 110
Thr Gly Arg Asn Trp Lys Gln Tyr Lys Gln Glu Asp Ser Ile Trp Thr
        115                 120                 125
Phe Ala Gly Asn Met Asn Ala Asp Ser Val Val His His Lys Leu Leu
    130                 135                 140
His Ser Val Arg Ala Arg Phe Val Arg Phe Val Pro Leu Glu Trp Asn
145                 150                 155                 160
Pro Ser Gly Lys Ile Gly Met Arg Val Glu Val Tyr Gly Cys Ser Tyr
                165                 170                 175
Lys Ser Asp Val Ala Asp Phe Asp Gly Arg Ser Ser Leu Leu Tyr Arg
            180                 185                 190
Phe Asn Gln Lys Leu Met Ser Thr Leu Lys Asp Val Ile Ser Leu Lys
        195                 200                 205
Phe Lys Ser Met Gln Gly Asp Gly Val Leu Phe His Gly Glu Gly Gln
    210                 215                 220
Arg Gly Asp His Ile Thr Leu Glu Leu Gln Lys Gly Arg Leu Ala Leu
225                 230                 235                 240
His Leu Asn Leu Gly Asp Ser Lys Ala Arg Leu Ser Thr Cys Pro Leu
                245                 250                 255
Pro Pro Trp Ala Ala Ser Trp Met Thr Ser Thr Gly Thr Xaa Ser Ser
            260                 265                 270
Leu Ser Gly Trp Ala Ser Arg
        275

<210> 11
<211> 1749
<212> DNA
<213> homo sapiens

<400> 11
   atggattctt taccacggct gaccagcgtt ttgactttgc tgttctctgg cttgtggcat 60
   ttaggattaa cagcgacaaa ctacaactgt gatgatccac tagcatccct gctctctcca 120
   atggcttttt ccagttcctc agacctcact ggcactcaca gcccagctca actcaactgg 180
   agagttggaa ctggcggttg gtccccagca gattccaatg ctcaacagtg gctccagatg 240
   gacctgggaa acagagtaga gattacagca gtggccacgc agggaagata cggaagctct 300
   gactgggtga cgagttacag cctgatgttc agtgacacag gacgcaactg gaaacagtac 360
   aaacaagaag acagcatctg gacctttgca ggaaacatga atgctgacag cgtggtgcac 420
   cacaagctat tgcactcagt gagagcccga tttgttcgct ttgtgcccct ggaatggaat 480
   cccagtggga agattggcat gagagtcgag gtctacggat gttcctataa atcagacgtt 540
   gctgactttg atggccgaag ctcacttctg tacaggttca atcagaagtt gatgagtact 600
   ctcaaagatg tgatctccct gaagttcaag agcatgcaag gagatggggt cctgttccat 660
   ggagaaggtc agcgtggaga ccacatcacc ttggaactcc agaaggggag gctcgcccta 720
   cacctcaatt tgggtgacag caaagcgcgg ctcagcagca gcttgccctc tgccaccctg 780
   ggcagcctcc tggatgacca gcactggcac tyggtcctca ttgagcgggt gggcaagcag 840
   gtgaacttca cggtggacaa gcacacacag cacttccgca ccaagggcga gacggatgcc 900
   ttagacattg actatgagct tagtttggga ggaattccag taccaggaaa acctgggacc 960
   tttttaaaga aaaacttcca tggatgcatc gaaaaccttt actacaatgg agtaaacata 1020
   attracctgg ctaagagacg aaagcatcag atctatactg tgggcaatgt cactttttcc 1080
   tgctccgaac cacagattgt gcccatcaca tttgtyaact ccagcggcag ctatttgctg 1140
   ctgcccggca ccccccaaat tgatgggctc tcagtgagtt tccagtttcg aacatggaac 1200
   aaggatggtc tgcttctgtc cacagagctg tctgagggct cgggaaccct gctgctgagc 1260
   ctggagggtg gaatcctgag actcgtgatt cagaaaatga cagaacgcgt agctgaaatc 1320
   ctcacaggca gcaacttgaa tgatggcctg tggcactcgg ttagcatcaa cgccaggagg 1380
   aaccgcatca cgctcactct ggatgatgaa gcagcacccc cggctccaga cagcacttgg 1440
   gtgcagattt attctggaaa tagctactat tttggaggtg tttgccaaac tactgtgaac 1500
   atggaggaag ctgctcccag tcctggacta ccttctattg taactgcagt gacacaagtt 1560
   acactggtgc cacctgccac aactccatct acgagcaatc ctgcgaggtg tacaggcacc 1620
   aggggaatac agccggcttc ttctacatcg actcagatgg cagcggccca ctgggacctc 1680
   tccaggtgta ctgcaatatc actgaggaca agatctggac atcagtgcag cacaacaata 1740
   cagagctga 1749

<210> 12
<211> 582
<212> PRT
<213> homo sapiens

<220>
<221> VARIANT
<222> 271, 342
<223> Xaa = Any Amino Acid

<400> 12
Met Asp Ser Leu Pro Arg Leu Thr Ser Val Leu Thr Leu Leu Phe Ser
1               5                   10                  15
Gly Leu Trp His Leu Gly Leu Thr Ala Thr Asn Tyr Asn Cys Asp Asp
            20                  25                  30
Pro Leu Ala Ser Leu Leu Ser Pro Met Ala Phe Ser Ser Ser Ser Asp
        35                  40                  45
Leu Thr Gly Thr His Ser Pro Ala Gln Leu Asn Trp Arg Val Gly Thr
    50                  55                  60
Gly Gly Trp Ser Pro Ala Asp Ser Asn Ala Gln Gln Trp Leu Gln Met
65                  70                  75                  80
Asp Leu Gly Asn Arg Val Glu Ile Thr Ala Val Ala Thr Gln Gly Arg
                85                  90                  95
Tyr Gly Ser Ser Asp Trp Val Thr Ser Tyr Ser Leu Met Phe Ser Asp
            100                 105                 110
Thr Gly Arg Asn Trp Lys Gln Tyr Lys Gln Glu Asp Ser Ile Trp Thr
        115                 120                 125
Phe Ala Gly Asn Met Asn Ala Asp Ser Val Val His His Lys Leu Leu
    130                 135                 140
His Ser Val Arg Ala Arg Phe Val Arg Phe Val Pro Leu Glu Trp Asn
145                 150                 155                 160
Pro Ser Gly Lys Ile Gly Met Arg Val Glu Val Tyr Gly Cys Ser Tyr
                165                 170                 175
Lys Ser Asp Val Ala Asp Phe Asp Gly Arg Ser Ser Leu Leu Tyr Arg
            180                 185                 190
Phe Asn Gln Lys Leu Met Ser Thr Leu Lys Asp Val Ile Ser Leu Lys
        195                 200                 205
Phe Lys Ser Met Gln Gly Asp Gly Val Leu Phe His Gly Glu Gly Gln
    210                 215                 220
Arg Gly Asp His Ile Thr Leu Glu Leu Gln Lys Gly Arg Leu Ala Leu
225                 230                 235                 240
His Leu Asn Leu Gly Asp Ser Lys Ala Arg Leu Ser Ser Ser Leu Pro
                245                 250                 255
Ser Ala Thr Leu Gly Ser Leu Leu Asp Asp Gln His Trp His Xaa Val
            260                 265                 270
Leu Ile Glu Arg Val Gly Lys Gln Val Asn Phe Thr Val Asp Lys His
        275                 280                 285

Thr Gln His Phe Arg Thr Lys Gly Glu Thr Asp Ala Leu Asp Ile Asp
    290                 295                 300
Tyr Glu Leu Ser Phe Gly Gly Ile Pro Val Pro Gly Lys Pro Gly Thr
305                 310                 315                 320
Phe Leu Lys Lys Asn Phe His Gly Cys Ile Glu Asn Leu Tyr Tyr Asn
                325                 330                 335
Gly Val Asn Ile Ile Xaa Leu Ala Lys Arg Arg Lys His Gln Ile Tyr
            340                 345                 350
Thr Val Gly Asn Val Thr Phe Ser Cys Ser Glu Pro Gln Ile Val Pro
        355                 360                 365
Ile Thr Phe Val Asn Ser Ser Gly Ser Tyr Leu Leu Leu Pro Gly Thr
    370                 375                 380
Pro Gln Ile Asp Gly Leu Ser Val Ser Phe Gln Phe Arg Thr Trp Asn
385                 390                 395                 400
Lys Asp Gly Leu Leu Leu Ser Thr Glu Leu Ser Glu Gly Ser Gly Thr
                405                 410                 415
Leu Leu Leu Ser Leu Glu Gly Gly Ile Leu Arg Leu Val Ile Gln Lys
            420                 425                 430
Met Thr Glu Arg Val Ala Glu Ile Leu Thr Gly Ser Asn Leu Asn Asp
        435                 440                 445
Gly Leu Trp His Ser Val Ser Ile Asn Ala Arg Arg Asn Arg Ile Thr
    450                 455                 460
Leu Thr Leu Asp Asp Glu Ala Ala Pro Pro Ala Pro Asp Ser Thr Trp
465                 470                 475                 480
Val Gln Ile Tyr Ser Gly Asn Ser Tyr Tyr Phe Gly Gly Val Cys Gln
                485                 490                 495
Thr Thr Val Asn Met Glu Glu Ala Ala Pro Ser Pro Gly Leu Pro Ser
            500                 505                 510
Ile Val Thr Ala Val Thr Gln Val Thr Leu Val Pro Pro Ala Thr Thr
        515                 520                 525
Pro Ser Thr Ser Asn Pro Ala Arg Cys Thr Gly Thr Arg Gly Ile Gln
    530                 535                 540
Pro Ala Ser Ser Thr Ser Thr Gln Met Ala Ala Ala His Trp Asp Leu
545                 550                 555                 560
Ser Arg Cys Thr Ala Ile Ser Leu Arg Thr Arg Ser Gly His Gln Cys
                565                 570                 575
Ser Thr Thr Ile Gln Ser
            580


<210> 13
<211> 1605
<212> DNA
<213> homo sapiens

<400> 13
atggattctt taccacggct gaccagcgtt ttgactttgc tgttctctgg cttgtggcat 60
ttaggattaa cagcgacaaa ctacaactgt gatgatccac tagcatccct gctctctcca 120
atggcttttt ccagttcctc agacctcact ggcactcaca gcccagctca actcaactgg 180
agagttggaa ctggcggttg gtccccagca gattccaatg ctcaacagtg gctccagatg 240
gacctgggaa acagagtaga gattacagca gtggccacgc agggaagata cggaagctct 300
gactgggtga cgagttacag cctgatgttc agtgacacag gacgcaactg gaaacagtac 360
aaacaagaag acagcatctg gacctttgca ggaaacatga atgctgacag cgtggtgcac 420
cacaagctat tgcactcagt gagagcccga tttgttcgct ttgtgcccct ggaatggaat 480
cccagtggga agattggcat gagagtcgag gtctacggat gttcctataa atcagacgtt 540
gctgactttg atggccgaag ctcacttctg tacaggttca atcagaagtt gatgagtact 600
ctcaaagatg tgatctccct gaagttcaag agcatgcaag gagatggggt cctgttccat 660
ggagaaggtc agcgtggaga ccacatcacc ttggaactcc agaaggggag gctcgcccta 720
cacctcaatt tgggtgacag caaagcgcgg ctcagcagca gcttgccctc tgccaccctg 780
ggcagcctcc tggatgacca gcactggcac tyggtcctca ttgagcgggt gggcaagcag 840
gtgaacttca cggtggacaa gcacacacag cacttccgca ccaagggcga gacggatgcc 900
ttagacattg actatgaggg caatgtcact ttttcctgct ccgaaccaca gattgtgccc 960
atcacatttg tyaactccag cggcagctat ttgctgctgc ccggcacccc ccaaattgat 1020
gggctctcag tgagtttcca gtttcgaaca tggaacaagg atggtctgct tctgtccaca 1080
gagctgtctg agggctcggg aaccctgctg ctgagcctgg agggtggaat cctgagactc 1140
gtgattcaga aaatgacaga acgcgtagct gaaatcctca caggcagcaa cttgaatgat 1200
ggcctgtggc actcggttag catcaacgcc aggaggaacc gcatcacgct cactctggat 1260
gatgaagcag cacccccggc tccagacagc acttgggtgc agatttattc tggaaatagc 1320

tactattttg gaggtgtttg ccaaactact gtgaacatgg aggaagctgc tcccagtcct 1380
ggactacctt ctattgtaac tgcagtgaca caagttacac tggtgccacc tgccacaact 1440
ccatctacga gcaatcctgc gaggtgtaca ggcaccaggg gaatacagcc ggcttcttct 1500
acatcgactc agatggcagc ggcccactgg gacctctcca ggtgtactgc aatatcactg 1560
aggacaagat ctggacatca gtgcagcaca acaatacaga gctga 1605

<210> 14
<211> 534
<212> PRT
<213> homo sapiens

<220>
<221> VARIANT
<222> 271
<223> Xaa = Any Amino Acid

<400> 14
Met Asp Ser Leu Pro Arg Leu Thr Ser Val Leu Thr Leu Leu Phe Ser
1               5                   10                  15
Gly Leu Trp His Leu Gly Leu Thr Ala Thr Asn Tyr Asn Cys Asp Asp
            20                  25                  30
Pro Leu Ala Ser Leu Leu Ser Pro Met Ala Phe Ser Ser Ser Ser Asp
        35                  40                  45
Leu Thr Gly Thr His Ser Pro Ala Gln Leu Asn Trp Arg Val Gly Thr
    50                  55                  60
Gly Gly Trp Ser Pro Ala Asp Ser Asn Ala Gln Gln Trp Leu Gln Met
65                  70                  75                  80
Asp Leu Gly Asn Arg Val Glu Ile Thr Ala Val Ala Thr Gln Gly Arg
                85                  90                  95
Tyr Gly Ser Ser Asp Trp Val Thr Ser Tyr Ser Leu Met Phe Ser Asp
            100                 105                 110
Thr Gly Arg Asn Trp Lys Gln Tyr Lys Gln Glu Asp Ser Ile Trp Thr
        115                 120                 125
Phe Ala Gly Asn Met Asn Ala Asp Ser Val Val His His Lys Leu Leu
    130                 135                 140
His Ser Val Arg Ala Arg Phe Val Arg Phe Val Pro Leu Glu Trp Asn
145                 150                 155                 160
Pro Ser Gly Lys Ile Gly Met Arg Val Glu Val Tyr Gly Cys Ser Tyr
                165                 170                 175
Lys Ser Asp Val Ala Asp Phe Asp Gly Arg Ser Ser Leu Leu Tyr Arg
            180                 185                 190
Phe Asn Gln Lys Leu Met Ser Thr Leu Lys Asp Val Ile Ser Leu Lys
        195                 200                 205
Phe Lys Ser Met Gln Gly Asp Gly Val Leu Phe His Gly Glu Gly Gln
    210                 215                 220
Arg Gly Asp His Ile Thr Leu Glu Leu Gln Lys Gly Arg Leu Ala Leu
225                 230                 235                 240
His Leu Asn Leu Gly Asp Ser Lys Ala Arg Leu Ser Ser Ser Leu Pro
                245                 250                 255
Ser Ala Thr Leu Gly Ser Leu Leu Asp Asp Gln His Trp His Xaa Val
            260                 265                 270
Leu Ile Glu Arg Val Gly Lys Gln Val Asn Phe Thr Val Asp Lys His
        275                 280                 285
Thr Gln His Phe Arg Thr Lys Gly Glu Thr Asp Ala Leu Asp Ile Asp
    290                 295                 300
Tyr Glu Gly Asn Val Thr Phe Ser Cys Ser Glu Pro Gln Ile Val Pro
305                 310                 315                 320
Ile Thr Phe Val Asn Ser Ser Gly Ser Tyr Leu Leu Leu Pro Gly Thr
                325                 330                 335
Pro Gln Ile Asp Gly Leu Ser Val Ser Phe Gln Phe Arg Thr Trp Asn
            340                 345                 350
Lys Asp Gly Leu Leu Leu Ser Thr Glu Leu Ser Glu Gly Ser Gly Thr
        355                 360                 365
Leu Leu Leu Ser Leu Glu Gly Gly Ile Leu Arg Leu Val Ile Gln Lys
    370                 375                 380
Met Thr Glu Arg Val Ala Glu Ile Leu Thr Gly Ser Asn Leu Asn Asp
385                 390                 395                 400
Gly Leu Trp His Ser Val Ser Ile Asn Ala Arg Arg Asn Arg Ile Thr

                    405                     410                     415
Leu Thr Leu Asp Asp Glu Ala Ala Pro Pro Ala Pro Asp Ser Thr Trp
            420                     425                     430
Val Gln Ile Tyr Ser Gly Asn Ser Tyr Tyr Phe Gly Gly Val Cys Gln
        435                     440                     445
Thr Thr Val Asn Met Glu Glu Ala Ala Pro Ser Pro Gly Leu Pro Ser
    450                     455                     460
Ile Val Thr Ala Val Thr Gln Val Thr Leu Val Pro Pro Ala Thr Thr
465                     470                     475                     480
Pro Ser Thr Ser Asn Pro Ala Arg Cys Thr Gly Thr Arg Gly Ile Gln
                    485                     490                     495
Pro Ala Ser Ser Thr Ser Thr Gln Met Ala Ala Ala His Trp Asp Leu
            500                     505                     510
Ser Arg Cys Thr Ala Ile Ser Leu Arg Thr Arg Ser Gly His Gln Cys
        515                     520                     525
Ser Thr Thr Ile Gln Ser
    530

<210> 15
<211> 2238
<212> DNA
<213> homo sapiens

<400> 15
atggattctt taccacggct gaccagcgtt ttgactttgc tgttctctgg cttgtggcat 60
ttaggattaa cagcgacaaa ctacaactgt gatgatccac tagcatccct gctctctcca 120
atggcttttt ccagttcctc agacctcact ggcactcaca gcccagctca actcaactgg 180
agagttggaa ctggcggttg gtccccagca gattccaatg ctcaacagtg gctccagatg 240
gacctgggaa acagagtaga gattacagca gtggccacgc agggaagata cggaagctct 300
gactgggtga cgagttacag cctgatgttc agtgacacag gacgcaactg gaaacagtac 360
aaacaagaag acagcatctg gacctttgca ggaaacatga atgctgacag cgtggtgcac 420
cacaagctat tgcactcagt gagagcccga tttgttcgct ttgtgcccct ggaatggaat 480
cccagtggga agattggcat gagagtcgag gtctacggat gttcctataa atcagacgtt 540
gctgactttg atggccgaag ctcacttctg tacaggttca atcagaagtt gatgagtact 600
ctcaaagatg tgatctccct gaagttcaag agcatgcaag gagatggggt cctgttccat 660
ggagaaggtc agcgtggaga ccacatcacc ttggaactcc agaaggggag gctcgcccta 720
cacctcaatt tgggtgacag caaagcgcgg ctcagcagca gcttgccctc tgccaccctg 780
ggcagcctcc tggatgacca gcactggcac tyggtcctca ttgagcgggt gggcaagcag 840
gtgaacttca cggtggacaa gcacacacag cacttccgca ccaagggcga gacggatgcc 900
ttagacattg actatgagct tagttttgga ggaattccag taccaggaaa acctgggacc 960
tttttaaaga aaaacttcca tggatgcatc gaaaaccttt actacaatgg agtaaacata 1020
attracctgg ctaagagacg aaagcatcag atctatactg tgggcaatgt cactttttcc 1080
tgctccgaac cacagattgt gcccatcaca tttgtyaact ccagcggcag ctatttgctg 1140
ctgcccggca ccccccaaat tgatgggctc tcagtgagtt tccagtttcg aacatggaac 1200
aaggatggtc tgcttctgtc cacagagctg tctgagggct cgggaaccct gctgctgagc 1260
ctggagggtg gaatcctgag actcgtgatt cagaaaatga cagaacgcgt agctgaaatc 1320
ctcacaggca gcaacttgaa tgatggcctg tggcactcgg ttagcatcaa cgccaggagg 1380
aaccgcatca cgctcactct ggatgatgaa gcagcacccc cggctccaga cagcacttgg 1440
gtgcagattt attctggaaa tagctactat tttggagggt gccccgacaa tctcaccgat 1500
tcccaatgtt taaatcccat taaggctttc caaggctgca tgaggctcat ctttattgat 1560
aaccagccca aggacctcat ttcagttcag caaggttccc tggggaattt tagtgattta 1620
cacattgatc tgtgtagcat caaagacagg tgtttgccaa actactgtga acatggagga 1680
agctgctccc agtcctggac taccttctat tgtaactgca gtgacacaag ttacactggt 1740
gccacctgcc acaactccat ctacgagcaa tcctgcgagg tgtacaggca ccaggggaat 1800
acagccggct tcttctacat cgactcagat ggcagcggcc cactgggacc tctccaggtg 1860
tactgcaata tcactgagga caagatctgg acatcagtgc agcacaacaa tacagagctg 1920
acccgagtgc ggggcgctaa ccctgagaag ccctatgcca tggccttgga ctacgggggc 1980
agcatggaac agctggaggc cgtgatcgac ggctctgagc actgtgagca ggaggtggcc 2040
taccactgca ggaggtcccg cctgctcaac acgccggatg gaacaccatt tacctggtgg 2100
attgggcggt ccaatgaaag gcacccttac tggggaggtt cccctcctgg ggtccagcag 2160
tgtgagtgtg gcctagacga gagctgcctg gacattcagc acttttgcaa ttgcgacgct 2220
gacaaggatg aatggtaa                                                2238

<210> 16
<211> 745
<212> PRT
<213> homo sapiens

<220>
<221> VARIANT
<222> 271, 342
<223> Xaa = Any Amino Acid

<400> 16
Met Asp Ser Leu Pro Arg Leu Thr Ser Val Leu Thr Leu Leu Phe Ser
1               5                   10                  15
Gly Leu Trp His Leu Gly Leu Thr Ala Thr Asn Tyr Asn Cys Asp Asp
            20                  25                  30
Pro Leu Ala Ser Leu Leu Ser Pro Met Ala Phe Ser Ser Ser Ser Asp
        35                  40                  45
Leu Thr Gly Thr His Ser Pro Ala Gln Leu Asn Trp Arg Val Gly Thr
    50                  55                  60
Gly Gly Trp Ser Pro Ala Asp Ser Asn Ala Gln Gln Trp Leu Gln Met
65                  70                  75                  80
Asp Leu Gly Asn Arg Val Glu Ile Thr Ala Val Ala Thr Gln Gly Arg
                85                  90                  95
Tyr Gly Ser Ser Asp Trp Val Thr Ser Tyr Ser Leu Met Phe Ser Asp
            100                 105                 110
Thr Gly Arg Asn Trp Lys Gln Tyr Lys Gln Glu Asp Ser Ile Trp Thr
        115                 120                 125
Phe Ala Gly Asn Met Asn Ala Asp Ser Val Val His His Lys Leu Leu
    130                 135                 140
His Ser Val Arg Ala Arg Phe Val Arg Phe Val Pro Leu Glu Trp Asn
145                 150                 155                 160
Pro Ser Gly Lys Ile Gly Met Arg Val Glu Val Tyr Gly Cys Ser Tyr
                165                 170                 175
Lys Ser Asp Val Ala Asp Phe Asp Gly Arg Ser Ser Leu Leu Tyr Arg
            180                 185                 190
Phe Asn Gln Lys Leu Met Ser Thr Leu Lys Asp Val Ile Ser Leu Lys
        195                 200                 205
Phe Lys Ser Met Gln Gly Asp Gly Val Leu Phe His Gly Glu Gly Gln
    210                 215                 220
Arg Gly Asp His Ile Thr Leu Glu Leu Gln Lys Gly Arg Leu Ala Leu
225                 230                 235                 240
His Leu Asn Leu Gly Asp Ser Lys Ala Arg Leu Ser Ser Ser Leu Pro
                245                 250                 255
Ser Ala Thr Leu Gly Ser Leu Leu Asp Asp Gln His Trp His Xaa Val
            260                 265                 270
Leu Ile Glu Arg Val Gly Lys Gln Val Asn Phe Thr Val Asp Lys His
        275                 280                 285
Thr Gln His Phe Arg Thr Lys Gly Glu Thr Asp Ala Leu Asp Ile Asp
    290                 295                 300
Tyr Glu Leu Ser Phe Gly Gly Ile Pro Val Pro Gly Lys Pro Gly Thr
305                 310                 315                 320
Phe Leu Lys Lys Asn Phe His Gly Cys Ile Glu Asn Leu Tyr Tyr Asn
                325                 330                 335
Gly Val Asn Ile Ile Xaa Leu Ala Lys Arg Arg Lys His Gln Ile Tyr
            340                 345                 350
Thr Val Gly Asn Val Thr Phe Ser Cys Ser Glu Pro Gln Ile Val Pro
        355                 360                 365
Ile Thr Phe Val Asn Ser Ser Gly Ser Tyr Leu Leu Leu Pro Gly Thr
    370                 375                 380
Pro Gln Ile Asp Gly Leu Ser Val Ser Phe Gln Phe Arg Thr Trp Asn
385                 390                 395                 400
Lys Asp Gly Leu Leu Leu Ser Thr Glu Leu Ser Glu Gly Ser Gly Thr
                405                 410                 415
Leu Leu Leu Ser Leu Glu Gly Gly Ile Leu Arg Leu Val Ile Gln Lys
            420                 425                 430
Met Thr Glu Arg Val Ala Glu Ile Leu Thr Gly Ser Asn Leu Asn Asp
        435                 440                 445
Gly Leu Trp His Ser Val Ser Ile Asn Ala Arg Arg Asn Arg Ile Thr
    450                 455                 460
Leu Thr Leu Asp Asp Glu Ala Ala Pro Pro Ala Pro Asp Ser Thr Trp
465                 470                 475                 480
Val Gln Ile Tyr Ser Gly Asn Ser Tyr Tyr Phe Gly Gly Cys Pro Asp

                485                 490                 495
Asn Leu Thr Asp Ser Gln Cys Leu Asn Pro Ile Lys Ala Phe Gln Gly
            500                 505                 510
Cys Met Arg Leu Ile Phe Ile Asp Asn Gln Pro Lys Asp Leu Ile Ser
        515                 520                 525
Val Gln Gln Gly Ser Leu Gly Asn Phe Ser Asp Leu His Ile Asp Leu
    530                 535                 540
Cys Ser Ile Lys Asp Arg Cys Leu Pro Asn Tyr Cys Glu His Gly Gly
545                 550                 555                 560
Ser Cys Ser Gln Ser Trp Thr Thr Phe Tyr Cys Asn Cys Ser Asp Thr
                565                 570                 575
Ser Tyr Thr Gly Ala Thr Cys His Asn Ser Ile Tyr Glu Gln Ser Cys
            580                 585                 590
Glu Val Tyr Arg His Gln Gly Asn Thr Ala Gly Phe Phe Tyr Ile Asp
        595                 600                 605
Ser Asp Gly Ser Gly Pro Leu Gly Pro Leu Gln Val Tyr Cys Asn Ile
    610                 615                 620
Thr Glu Asp Lys Ile Trp Thr Ser Val Gln His Asn Asn Thr Glu Leu
625                 630                 635                 640
Thr Arg Val Arg Gly Ala Asn Pro Glu Lys Pro Tyr Ala Met Ala Leu
                645                 650                 655
Asp Tyr Gly Gly Ser Met Glu Gln Leu Glu Ala Val Ile Asp Gly Ser
            660                 665                 670
Glu His Cys Glu Gln Glu Val Ala Tyr His Cys Arg Arg Ser Arg Leu
        675                 680                 685
Leu Asn Thr Pro Asp Gly Thr Pro Phe Thr Trp Trp Ile Gly Arg Ser
    690                 695                 700
Asn Glu Arg His Pro Tyr Trp Gly Gly Ser Pro Pro Gly Val Gln Gln
705                 710                 715                 720
Cys Glu Cys Gly Leu Asp Glu Ser Cys Leu Asp Ile Gln His Phe Cys
                725                 730                 735
Asn Cys Asp Ala Asp Lys Asp Glu Trp
            740                 745

<210> 17
<211> 2094
<212> DNA
<213> homo sapiens

<400> 17
   atggattctt taccacggct gaccagcgtt ttgactttgc tgttctctgg cttgtggcat 60
   ttaggattaa cagcgacaaa ctacaactgt gatgatccac tagcatccct gctctctcca 120
   atggcttttt ccagttcctc agacctcact ggcactcaca gcccagctca actcaactgg 180
   agagttggaa ctggcggttg gtccccagca gattccaatg ctcaacagtg gctccagatg 240
   gacctgggaa acagagtaga gattacagca gtggccacgc agggaagata cggaagctct 300
   gactgggtga cgagttacag cctgatgttc agtgacacag gacgcaactg gaaacagtac 360
   aaacaagaag acagcatctg gacctttgca ggaaacatga atgctgacag cgtggtgcac 420
   cacaagctat tgcactcagt gagagcccga tttgttcgct ttgtgcccct ggaatggaat 480
   cccagtggga agattggcat gagagtcgag gtctacggat gttcctataa atcagacgtt 540
   gctgactttg atggccgaag ctcacttctg tacaggttca atcagaagtt gatgagtact 600
   ctcaaagatg tgatctccct gaagttcaag agcatgcaag gagatggggt cctgttccat 660
   ggagaaggtc agcgtggaga ccacatcacc ttggaactcc agaaggggag gctcgcccta 720
   cacctcaatt tgggtgacag caaagcgcgg ctcagcagca gcttgccctc tgccaccctg 780
   ggcagcctcc tggatgacca gcactggcac tyggtcctca ttgagcgggt gggcaagcag 840
   gtgaacttca cggtggacaa gcacacacag cacttccgca ccaagggcga gacggatgcc 900
   ttagacattg actatgaggg caatgtcact ttttcctgct ccgaaccaca gattgtgccc 960
   atcacatttg tyaactccag cggcagctat ttgctgctgc ccggcacccc ccaaattgat 1020
   gggctctcag tgagtttcca gtttcgaaca tggaacaagg atggtctgct tctgtccaca 1080
   gagctgtctg agggctcggg aaccctgctg ctgagcctgg agggtggaat cctgagactc 1140
   gtgattcaga aaatgacaga acgcgtagct gaaatcctca caggcagcaa cttgaatgat 1200
   ggcctgtggc actcggttag catcaacgcc aggaggaacc gcatcacgct cactctggat 1260
   gatgaagcag cacccccggc tccagacagc acttgggtgc agatttattc tggaaatagc 1320
   tactattttg gagggtgccc cgacaatctc accgattccc aatgtttaaa tcccattaag 1380
   gctttccaag gctgcatgag gctcatcttt attgataacc agcccaagga cctcatttca 1440
   gttcagcaag gttccctggg gaattttagt gatttacaca ttgatctgtg tagcatcaaa 1500
   gacaggtgtt tgccaaacta ctgtgaacat ggaggaagct gctcccagtc ctggactacc 1560
   ttctattgta actgcagtga cacaagttac actggtgcca cctgccacaa ctccatctac 1620
   gagcaatcct gcgaggtgta caggcaccag gggaatacag ccggcttctt ctacatcgac 1680
   tcagatggca gcggcccact gggacctctc caggtgtact gcaatatcac tgaggacaag 1740
   atctggacat cagtgcagca caacaataca gagctgaccc gagtgcgggg cgctaaccct 1800
   gagaagccct atgccatggc cttggactac gggggcagca tggaacagct ggaggccgtg 1860
   atcgacggct ctgagcactg tgagcaggag gtggcctacc actgcaggag gtcccgcctg 1920
   ctcaacacgc cggatggaac accatttacc tggtggattg ggcggtccaa tgaaaggcac 1980
   ccttactggg gaggttcccc tcctggggtc cagcagtgtg agtgtggcct agacgagagc 2040
   tgcctggaca ttcagcactt ttgcaattgc gacgctgaca aggatgaatg gtaa 2094

<210> 18
<211> 697
<212> PRT
<213> homo sapiens

<220>
<221> VARIANT
<222> 271
<223> Xaa = Any Amino Acid

<400> 18
Met Asp Ser Leu Pro Arg Leu Thr Ser Val Leu Thr Leu Leu Phe Ser
1               5                   10                  15
Gly Leu Trp His Leu Gly Leu Thr Ala Thr Asn Tyr Asn Cys Asp Asp
            20                  25                  30
Pro Leu Ala Ser Leu Leu Ser Pro Met Ala Phe Ser Ser Ser Ser Asp
        35                  40                  45
Leu Thr Gly Thr His Ser Pro Ala Gln Leu Asn Trp Arg Val Gly Thr
    50                  55                  60
Gly Gly Trp Ser Pro Ala Asp Ser Asn Ala Gln Gln Trp Leu Gln Met
65                  70                  75                  80
Asp Leu Gly Asn Arg Val Glu Ile Thr Ala Val Ala Thr Gln Gly Arg
                85                  90                  95
Tyr Gly Ser Ser Asp Trp Val Thr Ser Tyr Ser Leu Met Phe Ser Asp
            100                 105                 110
Thr Gly Arg Asn Trp Lys Gln Tyr Lys Gln Glu Asp Ser Ile Trp Thr
        115                 120                 125
Phe Ala Gly Asn Met Asn Ala Asp Ser Val Val His His Lys Leu Leu
    130                 135                 140
His Ser Val Arg Ala Arg Phe Val Arg Phe Val Pro Leu Glu Trp Asn
145                 150                 155                 160
Pro Ser Gly Lys Ile Gly Met Arg Val Glu Val Tyr Gly Cys Ser Tyr
                165                 170                 175
Lys Ser Asp Val Ala Asp Phe Asp Gly Arg Ser Ser Leu Leu Tyr Arg
            180                 185                 190
Phe Asn Gln Lys Leu Met Ser Thr Leu Lys Asp Val Ile Ser Leu Lys
        195                 200                 205
Phe Lys Ser Met Gln Gly Asp Gly Val Leu Phe His Gly Glu Gly Gln
    210                 215                 220
Arg Gly Asp His Ile Thr Leu Glu Leu Gln Lys Gly Arg Leu Ala Leu
225                 230                 235                 240
His Leu Asn Leu Gly Asp Ser Lys Ala Arg Leu Ser Ser Ser Leu Pro
                245                 250                 255
Ser Ala Thr Leu Gly Ser Leu Leu Asp Asp Gln His Trp His Xaa Val
            260                 265                 270
Leu Ile Glu Arg Val Gly Lys Gln Val Asn Phe Thr Val Asp Lys His
        275                 280                 285
Thr Gln His Phe Arg Thr Lys Gly Glu Thr Asp Ala Leu Asp Ile Asp
    290                 295                 300
Tyr Glu Gly Asn Val Thr Phe Ser Cys Ser Glu Pro Gln Ile Val Pro
305                 310                 315                 320
Ile Thr Phe Val Asn Ser Ser Gly Ser Tyr Leu Leu Leu Pro Gly Thr
                325                 330                 335
Pro Gln Ile Asp Gly Leu Ser Val Ser Phe Gln Phe Arg Thr Trp Asn
            340                 345                 350
Lys Asp Gly Leu Leu Leu Ser Thr Glu Leu Ser Glu Gly Ser Gly Thr
        355                 360                 365
Leu Leu Leu Ser Leu Glu Gly Gly Ile Leu Arg Leu Val Ile Gln Lys
    370                 375                 380

Met Thr Glu Arg Val Ala Glu Ile Leu Thr Gly Ser Asn Leu Asn Asp
385                 390                 395                 400
Gly Leu Trp His Ser Val Ser Ile Asn Ala Arg Arg Asn Arg Ile Thr
                405                 410                 415
Leu Thr Leu Asp Asp Glu Ala Ala Pro Pro Ala Pro Asp Ser Thr Trp
            420                 425                 430
Val Gln Ile Tyr Ser Gly Asn Ser Tyr Tyr Phe Gly Gly Cys Pro Asp
        435                 440                 445
Asn Leu Thr Asp Ser Gln Cys Leu Asn Pro Ile Lys Ala Phe Gln Gly
    450                 455                 460
Cys Met Arg Leu Ile Phe Ile Asp Asn Gln Pro Lys Asp Leu Ile Ser
465                 470                 475                 480
Val Gln Gln Gly Ser Leu Gly Asn Phe Ser Asp Leu His Ile Asp Leu
                485                 490                 495
Cys Ser Ile Lys Asp Arg Cys Leu Pro Asn Tyr Cys Glu His Gly Gly
            500                 505                 510
Ser Cys Ser Gln Ser Trp Thr Thr Phe Tyr Cys Asn Cys Ser Asp Thr
        515                 520                 525
Ser Tyr Thr Gly Ala Thr Cys His Asn Ser Ile Tyr Glu Gln Ser Cys
    530                 535                 540
Glu Val Tyr Arg His Gln Gly Asn Thr Ala Gly Phe Phe Tyr Ile Asp
545                 550                 555                 560
Ser Asp Gly Ser Gly Pro Leu Gly Pro Leu Gln Val Tyr Cys Asn Ile
                565                 570                 575
Thr Glu Asp Lys Ile Trp Thr Ser Val Gln His Asn Asn Thr Glu Leu
            580                 585                 590
Thr Arg Val Arg Gly Ala Asn Pro Glu Lys Pro Tyr Ala Met Ala Leu
        595                 600                 605
Asp Tyr Gly Gly Ser Met Glu Gln Leu Glu Ala Val Ile Asp Gly Ser
    610                 615                 620
Glu His Cys Glu Gln Glu Val Ala Tyr His Cys Arg Arg Ser Arg Leu
625                 630                 635                 640
Leu Asn Thr Pro Asp Gly Thr Pro Phe Thr Trp Trp Ile Gly Arg Ser
                645                 650                 655
Asn Glu Arg His Pro Tyr Trp Gly Gly Ser Pro Pro Gly Val Gln Gln
            660                 665                 670
Cys Glu Cys Gly Leu Asp Glu Ser Cys Leu Asp Ile Gln His Phe Cys
        675                 680                 685
Asn Cys Asp Ala Asp Lys Asp Glu Trp
    690                 695

<210> 19
<211> 2520
<212> DNA
<213> homo sapiens

<400> 19
   atggattctt taccacggct gaccagcgtt ttgactttgc tgttctctgg cttgtggcat 60
   ttaggattaa cagcgacaaa ctacaactgt gatgatccac tagcatccct gctctctcca 120
   atggcttttt ccagttcctc agacctcact ggcactcaca gcccagctca actcaactgg 180
   agagttggaa ctggcggttg gtccccagca gattccaatg ctcaacagtg gctccagatg 240
   gacctgggaa acagagtaga gattacagca gtggccacgc agggaagata cggaagctct 300
   gactgggtga cgagttacag cctgatgttc agtgacacag gacgcaactg gaaacagtac 360
   aaacaagaag acagcatctg gacctttgca ggaaacatga atgctgacag cgtggtgcac 420
   cacaagctat tgcactcagt gagagcccga tttgttcgct ttgtgcccct ggaatggaat 480
   cccagtggga agattggcat gagagtcgag gtctacggat gttcctataa atcagacgtt 540
   gctgactttg atggccgaag ctcacttctg tacaggttca atcagaagtt gatgagtact 600
   ctcaaagatg tgatctccct gaagttcaag agcatgcaag gagatggggt cctgttccat 660
   ggagaaggtc agcgtggaga ccacatcacc ttggaactcc agaaggggag gctcgcccta 720
   cacctcaatt tgggtgacag caaagcgcgg ctcagcagca gcttgccctc tgccaccctg 780
   ggcagcctcc tggatgacca gcactggcac tyggtcctca ttgagcgggt gggcaagcag 840
   gtgaacttca cggtggacaa gcacacacag cacttccgca ccaagggcga gacggatgcc 900
   ttagacattg actatgagct tagttttgga ggaattccag taccaggaaa acctgggacc 960
   tttttaaaga aaaacttcca tggatgcatc gaaaaccttt actacaatgg agtaaacata 1020
   attracctgg ctaagagacg aaagcatcag atctatactg tgggcaatgt cactttttcc 1080
   tgctccgaac cacagattgt gcccatcaca tttgtyaact ccagcggcag ctatttgctg 1140
   ctgcccggca ccccccaaat tgatgggctc tcagtgagtt tccagtttcg aacatggaac 1200
   aaggatggtc tgcttctgtc cacagagctg tctgagggct cgggaaccct gctgctgagc 1260
   ctggagggtg gaatcctgag actcgtgatt cagaaaatga cagaacgcgt agctgaaatc 1320
   ctcacaggca gcaacttgaa tgatggcctg tggcactcgg ttagcatcaa cgccaggagg 1380
   aaccgcatca cgctcactct ggatgatgaa gcagcacccc cggctccaga cagcacttgg 1440
   gtgcagattt attctggaaa tagctactat tttggagggt gccccgacaa tctcaccgat 1500
   tcccaatgtt taaatcccat taaggctttc caaggctgca tgaggctcat ctttattgat 1560
   aaccagccca aggacctcat ttcagttcag caaggttccc tggggaattt tagtgattta 1620
   cacattgatc tgtgtagcat caaagacagg tgtttgccaa actactgtga acatggagga 1680
   agctgctccc agtcctggac taccttctat tgtaactgca gtgacacaag ttacactggt 1740
   gccacctgcc acaactccat ctacgagcaa tcctgcgagg tgtacaggca ccaggggaat 1800
   acagccggct tcttctacat cgactcagat ggcagcggcc cactgggacc tctccaggtg 1860
   tactgcaata tcactgagga caagatctgg acatcagtgc agcacaacaa tacagagctg 1920
   acccgagtgc ggggcgctaa ccctgagaag ccctatgcca tggccttgga ctacgggggc 1980
   agcatggaac agctggaggc cgtgatcgac ggctctgagc actgtgagca ggaggtggcc 2040
   taccactgca ggaggtcccg cctgctcaac acgccggatg gaacaccatt tacctggtgg 2100
   attgggcggt ccaatgaaag gcacccttac tggggaggtt cccctcctgg ggtccagcag 2160
   tgtgagtgtg gcctagacga gagctgcctg gacattcagc acttttgcaa ttgcgacgct 2220
   gacaaggatg aatggacaaa tgatactggc tttctttcct tcaaagacca cttgcctgtc 2280
   actcagatag ttatcactga taccgacaga tcaaactcag aagccgcttg gagaattggt 2340
   cccttgcgtt gctatggtga ccgtgagtac aaaatcgaaa gaagctttct ctctgcatta 2400
   catgagcaca agatgttctt actcccttat cccttttccc tgcagtgtgc cctagtcttg 2460
   aaaattatcc acatgtccag tgctttccca taccccactg aaaacgataa accatgttga 2520

<210> 20
<211> 839
<212> PRT
<213> homo sapiens

<220>
<221> VARIANT
<222> 271, 342
<223> Xaa = Any Amino Acid

<400> 20
Met Asp Ser Leu Pro Arg Leu Thr Ser Val Leu Thr Leu Leu Phe Ser
1               5                   10                  15
Gly Leu Trp His Leu Gly Leu Thr Ala Thr Asn Tyr Asn Cys Asp Asp
            20                  25                  30
Pro Leu Ala Ser Leu Leu Ser Pro Met Ala Phe Ser Ser Ser Ser Asp
        35                  40                  45
Leu Thr Gly Thr His Ser Pro Ala Gln Leu Asn Trp Arg Val Gly Thr
    50                  55                  60
Gly Gly Trp Ser Pro Ala Asp Ser Asn Ala Gln Gln Trp Leu Gln Met
65                  70                  75                  80
Asp Leu Gly Asn Arg Val Glu Ile Thr Ala Val Ala Thr Gln Gly Arg
                85                  90                  95
Tyr Gly Ser Ser Asp Trp Val Thr Ser Tyr Ser Leu Met Phe Ser Asp
            100                 105                 110
Thr Gly Arg Asn Trp Lys Gln Tyr Lys Gln Glu Asp Ser Ile Trp Thr
        115                 120                 125
Phe Ala Gly Asn Met Asn Ala Asp Ser Val Val His His Lys Leu Leu
    130                 135                 140
His Ser Val Arg Ala Arg Phe Val Arg Phe Val Pro Leu Glu Trp Asn
145                 150                 155                 160
Pro Ser Gly Lys Ile Gly Met Arg Val Glu Val Tyr Gly Cys Ser Tyr
                165                 170                 175
Lys Ser Asp Val Ala Asp Phe Asp Gly Arg Ser Ser Leu Leu Tyr Arg
            180                 185                 190
Phe Asn Gln Lys Leu Met Ser Thr Leu Lys Asp Val Ile Ser Leu Lys
        195                 200                 205
Phe Lys Ser Met Gln Gly Asp Gly Val Leu Phe His Gly Glu Gly Gln
    210                 215                 220
Arg Gly Asp His Ile Thr Leu Glu Leu Gln Lys Gly Arg Leu Ala Leu
225                 230                 235                 240
His Leu Asn Leu Gly Asp Ser Lys Ala Arg Leu Ser Ser Ser Leu Pro
                245                 250                 255
Ser Ala Thr Leu Gly Ser Leu Leu Asp Asp Gln His Trp His Xaa Val

            260                 265                 270
Leu Ile Glu Arg Val Gly Lys Gln Val Asn Phe Thr Val Asp Lys His
        275                 280                 285
Thr Gln His Phe Arg Thr Lys Gly Glu Thr Asp Ala Leu Asp Ile Asp
    290                 295                 300
Tyr Glu Leu Ser Phe Gly Gly Ile Pro Val Pro Gly Lys Pro Gly Thr
305                 310                 315                 320
Phe Leu Lys Lys Asn Phe His Gly Cys Ile Glu Asn Leu Tyr Tyr Asn
                325                 330                 335
Gly Val Asn Ile Ile Xaa Leu Ala Lys Arg Arg Lys His Gln Ile Tyr
            340                 345                 350
Thr Val Gly Asn Val Thr Phe Ser Cys Ser Glu Pro Gln Ile Val Pro
        355                 360                 365
Ile Thr Phe Val Asn Ser Ser Gly Ser Tyr Leu Leu Leu Pro Gly Thr
    370                 375                 380
Pro Gln Ile Asp Gly Leu Ser Val Ser Phe Gln Phe Arg Thr Trp Asn
385                 390                 395                 400
Lys Asp Gly Leu Leu Leu Ser Thr Glu Leu Ser Glu Gly Ser Gly Thr
                405                 410                 415
Leu Leu Leu Ser Leu Glu Gly Gly Ile Leu Arg Leu Val Ile Gln Lys
            420                 425                 430
Met Thr Glu Arg Val Ala Glu Ile Leu Thr Gly Ser Asn Leu Asn Asp
        435                 440                 445
Gly Leu Trp His Ser Val Ser Ile Asn Ala Arg Arg Asn Arg Ile Thr
    450                 455                 460
Leu Thr Leu Asp Asp Glu Ala Ala Pro Pro Ala Pro Asp Ser Thr Trp
465                 470                 475                 480
Val Gln Ile Tyr Ser Gly Asn Ser Tyr Tyr Phe Gly Gly Cys Pro Asp
                485                 490                 495
Asn Leu Thr Asp Ser Gln Cys Leu Asn Pro Ile Lys Ala Phe Gln Gly
            500                 505                 510
Cys Met Arg Leu Ile Phe Ile Asp Asn Gln Pro Lys Asp Leu Ile Ser
        515                 520                 525
Val Gln Gln Gly Ser Leu Gly Asn Phe Ser Asp Leu His Ile Asp Leu
    530                 535                 540
Cys Ser Ile Lys Asp Arg Cys Leu Pro Asn Tyr Cys Glu His Gly Gly
545                 550                 555                 560
Ser Cys Ser Gln Ser Trp Thr Thr Phe Tyr Cys Asn Cys Ser Asp Thr
                565                 570                 575
Ser Tyr Thr Gly Ala Thr Cys His Asn Ser Ile Tyr Glu Gln Ser Cys
            580                 585                 590
Glu Val Tyr Arg His Gln Gly Asn Thr Ala Gly Phe Phe Tyr Ile Asp
        595                 600                 605
Ser Asp Gly Ser Gly Pro Leu Gly Pro Leu Gln Val Tyr Cys Asn Ile
    610                 615                 620
Thr Glu Asp Lys Ile Trp Thr Ser Val Gln His Asn Asn Thr Glu Leu
625                 630                 635                 640
Thr Arg Val Arg Gly Ala Asn Pro Glu Lys Pro Tyr Ala Met Ala Leu
                645                 650                 655
Asp Tyr Gly Gly Ser Met Glu Gln Leu Glu Ala Val Ile Asp Gly Ser
            660                 665                 670
Glu His Cys Glu Gln Glu Val Ala Tyr His Cys Arg Arg Ser Arg Leu
        675                 680                 685
Leu Asn Thr Pro Asp Gly Thr Pro Phe Thr Trp Trp Ile Gly Arg Ser
    690                 695                 700
Asn Glu Arg His Pro Tyr Trp Gly Gly Ser Pro Pro Gly Val Gln Gln
705                 710                 715                 720
Cys Glu Cys Gly Leu Asp Glu Ser Cys Leu Asp Ile Gln His Phe Cys
                725                 730                 735
Asn Cys Asp Ala Asp Lys Asp Glu Trp Thr Asn Asp Thr Gly Phe Leu
            740                 745                 750
Ser Phe Lys Asp His Leu Pro Val Thr Gln Ile Val Ile Thr Asp Thr
        755                 760                 765
Asp Arg Ser Asn Ser Glu Ala Ala Trp Arg Ile Gly Pro Leu Arg Cys
    770                 775                 780
Tyr Gly Asp Arg Glu Tyr Lys Ile Glu Arg Ser Phe Leu Ser Ala Leu
785                 790                 795                 800
His Glu His Lys Met Phe Leu Leu Pro Tyr Pro Phe Ser Leu Gln Cys

805                    810                    815
Ala Leu Val Leu Lys Ile Ile His Met Ser Ser Ala Phe Pro Tyr Pro
            820                    825                    830
Thr Glu Asn Asp Lys Pro Cys
        835

<210> 21
<211> 2376
<212> DNA
<213> homo sapiens

<400> 21
   atggattctt taccacggct gaccagcgtt ttgactttgc tgttctctgg cttgtggcat 60
   ttaggattaa cagcgacaaa ctacaactgt gatgatccac tagcatccct gctctctcca 120
   atggcttttt ccagttcctc agacctcact ggcactcaca gcccagctca actcaactgg 180
   agagttggaa ctggcggttg gtccccagca gattccaatg ctcaacagtg gctccagatg 240
   gacctgggaa acagagtaga gattacagca gtggccacgc agggaagata cggaagctct 300
   gactgggtga cgagttacag cctgatgttc agtgacacag gacgcaactg gaaacagtac 360
   aaacaagaag acagcatctg gacctttgca ggaaacatga atgctgacag cgtggtgcac 420
   cacaagctat tgcactcagt gagagcccga tttgttcgct ttgtgcccct ggaatggaat 480
   cccagtggga agattggcat gagagtcgag gtctacggat gttcctataa atcagacgtt 540
   gctgactttg atggccgaag ctcacttctg tacaggttca atcagaagtt gatgagtact 600
   ctcaaagatg tgatctccct gaagttcaag agcatgcaag gagatggggt cctgttccat 660
   ggagaaggtc agcgtggaga ccacatcacc ttggaactcc agaaggggag gctcgcccta 720
   cacctcaatt tgggtgacag caaagcgcgg ctcagcagca gcttgccctc tgccaccctg 780
   ggcagcctcc tggatgacca gcactggcac tyggtcctca ttgagcgggt gggcaagcag 840
   gtgaacttca cggtggacaa gcacacacag cacttccgca ccaagggcga gacggatgcc 900
   ttagacattg actatgaggg caatgtcact ttttcctgct ccgaaccaca gattgtgccc 960
   atcacatttg tyaactccag cggcagctat ttgctgctgc ccggcacccc ccaaattgat 1020
   gggctctcag tgagtttcca gtttcgaaca tggaacaagg atggtctgct tctgtccaca 1080
   gagctgtctg agggctcggg aaccctgctg ctgagcctgg agggtggaat cctgagactc 1140
   gtgattcaga aaatgacaga acgcgtagct gaaatcctca caggcagcaa cttgaatgat 1200
   ggcctgtggc actcggttag catcaacgcc aggaggaacc gcatcacgct cactctggat 1260
   gatgaagcag cacccccggc tccagacagc acttgggtgc agatttattc tggaaatagc 1320
   tactattttg gagggtgccc cgacaatctc accgattccc aatgtttaaa tcccattaag 1380
   gctttccaag gctgcatgag gctcatcttt attgataacc agcccaagga cctcatttca 1440
   gttcagcaag gttccctggg gaattttagt gatttacaca ttgatctgtg tagcatcaaa 1500
   gacaggtgtt tgccaaacta ctgtgaacat ggaggaagct gctcccagtc ctggactacc 1560
   ttctattgta actgcagtga cacaagttac actggtgcca cctgccacaa ctccatctac 1620
   gagcaatcct gcgaggtgta caggcaccag gggaatacag ccggcttctt ctacatcgac 1680
   tcagatggca gcggcccact gggacctctc caggtgtact gcaatatcac tgaggacaag 1740
   atctggacat cagtgcagca caacaataca gagctgaccc gagtgcgggg cgctaaccct 1800
   gagaagccct atgccatggc cttggactac gggggcagca tggaacagct ggaggccgtg 1860
   atcgacggct ctgagcactg tgagcaggag gtggcctacc actgcaggag gtcccgcctg 1920
   ctcaacacgc cggatggaac accatttacc tggtggattg ggcggtccaa tgaaaggcac 1980
   ccttactggg gaggttcccc tcctggggtc cagcagtgtg agtgtggcct agacgagagc 2040
   tgcctggaca ttcagcactt ttgcaattgc gacgctgaca aggatgaatg gacaaatgat 2100
   actggctttc tttccttcaa agaccacttg cctgtcactc agatagttat cactgatacc 2160
   gacagatcaa actcagaagc cgcttggaga attggtccct tgcgttgcta tggtgaccgt 2220
   gagtacaaaa tcgaaagaag ctttctctct gcattacatg agcacaagat gttcttactc 2280
   ccttatccct tttccctgca gtgtgcccta gtcttgaaaa ttatccacat gtccagtgct 2340
   ttcccatacc ccactgaaaa cgataaacca tgttga 2376

<210> 22
<211> 791
<212> PRT
<213> homo sapiens

<220>
<221> VARIANT
<222> 271
<223> Xaa = Any Amino Acid

<400> 22
Met Asp Ser Leu Pro Arg Leu Thr Ser Val Leu Thr Leu Leu Phe Ser
 1               5                   10                  15
Gly Leu Trp His Leu Gly Leu Thr Ala Thr Asn Tyr Asn Cys Asp Asp

20                  25                  30
Pro Leu Ala Ser Leu Leu Ser Pro Met Ala Phe Ser Ser Ser Ser Asp
        35                  40                  45
Leu Thr Gly Thr His Ser Pro Ala Gln Leu Asn Trp Arg Val Gly Thr
    50                  55                  60
Gly Gly Trp Ser Pro Ala Asp Ser Asn Ala Gln Gln Trp Leu Gln Met
65                  70                  75                  80
Asp Leu Gly Asn Arg Val Glu Ile Thr Ala Val Ala Thr Gln Gly Arg
                85                  90                  95
Tyr Gly Ser Ser Asp Trp Val Thr Ser Tyr Ser Leu Met Phe Ser Asp
            100                 105                 110
Thr Gly Arg Asn Trp Lys Gln Tyr Lys Gln Glu Asp Ser Ile Trp Thr
        115                 120                 125
Phe Ala Gly Asn Met Asn Ala Asp Ser Val Val His His Lys Leu Leu
    130                 135                 140
His Ser Val Arg Ala Arg Phe Val Arg Phe Val Pro Leu Glu Trp Asn
145                 150                 155                 160
Pro Ser Gly Lys Ile Gly Met Arg Val Glu Val Tyr Gly Cys Ser Tyr
                165                 170                 175
Lys Ser Asp Val Ala Asp Phe Asp Gly Arg Ser Ser Leu Leu Tyr Arg
            180                 185                 190
Phe Asn Gln Lys Leu Met Ser Thr Leu Lys Asp Val Ile Ser Leu Lys
        195                 200                 205
Phe Lys Ser Met Gln Gly Asp Gly Val Leu Phe His Gly Glu Gly Gln
    210                 215                 220
Arg Gly Asp His Ile Thr Leu Glu Leu Gln Lys Gly Arg Leu Ala Leu
225                 230                 235                 240
His Leu Asn Leu Gly Asp Ser Lys Ala Arg Leu Ser Ser Ser Leu Pro
                245                 250                 255
Ser Ala Thr Leu Gly Ser Leu Leu Asp Asp Gln His Trp His Xaa Val
            260                 265                 270
Leu Ile Glu Arg Val Gly Lys Gln Val Asn Phe Thr Val Asp Lys His
        275                 280                 285
Thr Gln His Phe Arg Thr Lys Gly Glu Thr Asp Ala Leu Asp Ile Asp
    290                 295                 300
Tyr Glu Gly Asn Val Thr Phe Ser Cys Ser Glu Pro Gln Ile Val Pro
305                 310                 315                 320
Ile Thr Phe Val Asn Ser Ser Gly Ser Tyr Leu Leu Leu Pro Gly Thr
                325                 330                 335
Pro Gln Ile Asp Gly Leu Ser Val Ser Phe Gln Phe Arg Thr Trp Asn
            340                 345                 350
Lys Asp Gly Leu Leu Leu Ser Thr Glu Leu Ser Glu Gly Ser Gly Thr
        355                 360                 365
Leu Leu Leu Ser Leu Glu Gly Gly Ile Leu Arg Leu Val Ile Gln Lys
    370                 375                 380
Met Thr Glu Arg Val Ala Glu Ile Leu Thr Gly Ser Asn Leu Asn Asp
385                 390                 395                 400
Gly Leu Trp His Ser Val Ser Ile Asn Ala Arg Arg Asn Arg Ile Thr
                405                 410                 415
Leu Thr Leu Asp Asp Glu Ala Ala Pro Pro Ala Pro Asp Ser Thr Trp
            420                 425                 430
Val Gln Ile Tyr Ser Gly Asn Ser Tyr Tyr Phe Gly Gly Cys Pro Asp
        435                 440                 445
Asn Leu Thr Asp Ser Gln Cys Leu Asn Pro Ile Lys Ala Phe Gln Gly
    450                 455                 460
Cys Met Arg Leu Ile Phe Ile Asp Asn Gln Pro Lys Asp Leu Ile Ser
465                 470                 475                 480
Val Gln Gln Gly Ser Leu Gly Asn Phe Ser Asp Leu His Ile Asp Leu
                485                 490                 495
Cys Ser Ile Lys Asp Arg Cys Leu Pro Asn Tyr Cys Glu His Gly Gly
            500                 505                 510
Ser Cys Ser Gln Ser Trp Thr Thr Phe Tyr Cys Asn Cys Ser Asp Thr
        515                 520                 525
Ser Tyr Thr Gly Ala Thr Cys His Asn Ser Ile Tyr Glu Gln Ser Cys
    530                 535                 540
Glu Val Tyr Arg His Gln Gly Asn Thr Ala Gly Phe Phe Tyr Ile Asp
545                 550                 555                 560
Ser Asp Gly Ser Gly Pro Leu Gly Pro Leu Gln Val Tyr Cys Asn Ile

565                     570                     575
Thr Glu Asp Lys Ile Trp Thr Ser Val Gln His Asn Asn Thr Glu Leu
            580                     585                     590
Thr Arg Val Arg Gly Ala Asn Pro Glu Lys Pro Tyr Ala Met Ala Leu
        595                     600                     605
Asp Tyr Gly Gly Ser Met Glu Gln Leu Glu Ala Val Ile Asp Gly Ser
    610                     615                     620
Glu His Cys Glu Gln Glu Val Ala Tyr His Cys Arg Arg Ser Arg Leu
625                     630                     635                     640
Leu Asn Thr Pro Asp Gly Thr Pro Phe Thr Trp Trp Ile Gly Arg Ser
                645                     650                     655
Asn Glu Arg His Pro Tyr Trp Gly Gly Ser Pro Pro Gly Val Gln Gln
            660                     665                     670
Cys Glu Cys Gly Leu Asp Glu Ser Cys Leu Asp Ile Gln His Phe Cys
        675                     680                     685
Asn Cys Asp Ala Asp Lys Asp Glu Trp Thr Asn Asp Thr Gly Phe Leu
    690                     695                     700
Ser Phe Lys Asp His Leu Pro Val Thr Gln Ile Val Ile Thr Asp Thr
705                     710                     715                     720
Asp Arg Ser Asn Ser Glu Ala Ala Trp Arg Ile Gly Pro Leu Arg Cys
                725                     730                     735
Tyr Gly Asp Arg Glu Tyr Lys Ile Glu Arg Ser Phe Leu Ser Ala Leu
            740                     745                     750
His Glu His Lys Met Phe Leu Leu Pro Tyr Pro Phe Ser Leu Gln Cys
        755                     760                     765
Ala Leu Val Leu Lys Ile Ile His Met Ser Ser Ala Phe Pro Tyr Pro
    770                     775                     780
Thr Glu Asn Asp Lys Pro Cys
785                     790

<210> 23
<211> 3897
<212> DNA
<213> homo sapiens

<400> 23
atgtttcttg ctaatagaat ctggtctctc tccctctgtc ctcctatcat tatagacaac 60
tgtgatgatc cactagcatc cctgctctct ccaatggctt tttccagttc ctcagacctc 120
actggcactc acagcccagc tcaactcaac tggagagttg gaactggcgg ttggtcccca 180
gcagattcca atgctcaaca gtggctccag atggacctgg gaaacagagt agagattaca 240
gcagtggcca cgcagggaag atacggaagc tctgactggg tgacgagtta cagcctgatg 300
ttcagtgaca caggacgcaa ctggaaacag tacaaacaag aagacagcat ctggaccttt 360
gcaggaaaca tgaatgctga cagcgtggtg caccacaagc tattgcactc agtgagagcc 420
cgatttgttc gctttgtgcc cctggaatgg aatcccagtg ggaagattgg catgagagtc 480
gaggtctacg gatgttccta taaatcagac gttgctgact ttgatggccg aagctcactt 540
ctgtacaggt tcaatcagaa gttgatgagt actctcaaag atgtgatctc cctgaagttc 600
aagagcatgc aaggagatgg ggtcctgttc catggagaag gtcagcgtgg agaccacatc 660
accttggaac tccagaaggg gaggctcgcc ctacacctca atttgggtga cagcaaagcg 720
cggctcagca gcagcttgcc ctctgccacc ctgggcagcc tcctggatga ccagcactgg 780
cactcggtcc tcattgagcg ggtgggcaag caggtgaact tcacggtgga caagcacaca 840
cagcacttcc gcaccaaggg cgagacggat gccttagaca ttgactatga gcttagtttt 900
ggaggaattc cagtaccagg aaaacctggg acctttttaa agaaaaactt ccatggatgc 960
atcgaaaacc tttactacaa tggagtaaac ataattgacc tggctaagag acgaaagcat 1020
cagatctata ctgtgggcaa tgtcactttt tcctgctccg aaccacagat tgtgcccatc 1080
acatttgtca actccagcgg cagctatttg ctgctgcccg gcacccccca aattgatggg 1140
ctctcagtga gtttccagtt tcgaacatgg aacaaggatg gtctgcttct gtccacagag 1200
ctgtctgagg gctcgggaac cctgctgctg agcctggagg gtggaatcct gagactcgtg 1260
attcagaaaa tgacagaacg cgtagctgaa atcctcacag gcagcaactt gaatgatggc 1320
ctgtggcact cggttagcat caacgccagg aggaaccgca tcacgctcac tctggatgat 1380
gaagcagcac ccccggctcc agacagcact tgggtgcaga tttattctgg aaatagctac 1440
tattttggag ggtgccccga caatctcacc gattcccaat gtttaaatcc cattaaggct 1500
ttccaaggct gcatgaggct catctttatt gataaccagc ccaaggacct catttcagtt 1560
cagcaaggtt ccctggggaa ttttagtgat ttacacattg atctgtgtag catcaaagac 1620
aggtgtttgc caaactactg tgaacatgga ggaagctgct cccagtcctg gactaccttc 1680
tattgtaact gcagtgacac aagttacact ggtgccacct gccacaactc catctacgag 1740
caatcctgcg aggtgtacag gcaccagggg aatacagccg gcttcttcta catcgactca 1800
gatggcagcg gcccactggg acctctccag gtgtactgca atatcactga ggacaagatc 1860

tggacatcag tgcagcacaa caatacagag ctgacccgag tgcggggcgc taaccctgag 1920
aagccctatg ccatggcctt ggactacggg ggcagcatgg aacagctgga ggccgtgatc 1980
gacggctctg agcactgtga gcaggaggtg gcctaccact gcaggaggtc ccgcctgctc 2040
aacacgccgg atggaacacc atttacctgg tggattgggc ggtccaatga aaggcaccct 2100
tactggggag gttcccctcc tggggtccag cagtgtgagt gtggcctaga cgagagctgc 2160
ctggacattc agcacttttg caattgcgac gctgacaagg atgaatggac aaatgatact 2220
ggctttcttt ccttcaaaga ccacttgcct gtcactcaga tagttatcac tgataccgac 2280
agatcaaact cagaagccgc ttggagaatt ggtcccttgc gttgctatgg tgaccgacgc 2340
ttctggaacg ccgtctcatt ttatacagaa gcctcttacc tccactttcc taccttccat 2400
gcggaattca gtgccgatat ttccttcttt tttaaaacca cagcattatc cggagttttc 2460
ctagaaaatc ttggcattaa agacttcatt cgactcgaaa taagctctcc ttcagagatc 2520
acctttgcca tcgatgttgg gaatggtcct gtggagcttg tagtccagtc tccttctctt 2580
ctgaatgaca accaatggca ctatgtccgg gctgagagga acctcaagga gacctccctg 2640
caggtggaca accttccaag gagcaccagg gagacgtcgg aggagggcca ttttcgactg 2700
cagctgaaca gccagttgtt tgtaggggga acgtcatcca gacagaaagg cttcctagga 2760
tgcattcgct ccttacactt gaatggacag aaaatggacc tggaagagag ggcaaaggtc 2820
acatctggag tcaggccagg ctgccccggc cactgcagca gctacggcag catctgccac 2880
aacgggggca agtgtgtgga gaagcacaat ggctacctgt gtgattgcac caattcacct 2940
tatgaagggc ccttttgcaa aaaagaggtt tctgctgttt ttgaggctgg cacgtcggtt 3000
acttacatgt ttcaagaacc ctatcctgtg accaagaata taagcctctc atcctcagct 3060
atttacacag attcagctcc atccaaggaa aacattgcac ttagctttgt gacaacccag 3120
gcacccagtc ttttgctctt tatcaattct tcttctcagg acttcgtggt tgttctgctc 3180
tgcaagaatg gaagcttaca ggttcgctat cacctaaaca aggaagaaac ccatgtattc 3240
accattgatg cagataactt tgctaacaga aggatgcacc acttgaagat taaccgagag 3300
ggaagagagc ttaccattca gatggaccag caacttcgac tcagttataa cttctctccg 3360
gaagtagagt tcagggttat aaggtcactc accttgggca aagtcacaga gaatcttggt 3420
ttggattctg aagttgctaa agcaaatgcc atgggttttg ctggatgcat gtcttccgtc 3480
cagtacaacc acatagcacc actgaaggct gccctgcgcc atgccactgt cgcgcctgtg 3540
actgtccatg ggaccttgac ggaatccagc tgtggcttca tggtggactc agatgtgaat 3600
gcagtgacca cggtgcattc ttcatcagat ccttttggga agacagatga gcgggaacca 3660
ctcacaaatg ctgttcgaag tgattcggca gtcatcggag gggtgatagc agtggtgata 3720
ttcatcatct tctgtatcat cggcatcatg acccggttcc tctaccagca caagcagtca 3780
catcgtacga gccagatgaa ggagaaggaa tatccagaaa atttggacag ttccttcaga 3840
aatgaaattg acttgcaaaa cacagtgagc gagtgtaaac gggaatattt catctga 3897

<210> 24
<211> 1298
<212> PRT
<213> homo sapiens

<400> 24
Met Phe Leu Ala Asn Arg Ile Trp Ser Leu Ser Leu Cys Pro Pro Ile
1               5                   10                  15
Ile Ile Asp Asn Cys Asp Asp Pro Leu Ala Ser Leu Leu Ser Pro Met
            20                  25                  30
Ala Phe Ser Ser Ser Ser Asp Leu Thr Gly Thr His Ser Pro Ala Gln
        35                  40                  45
Leu Asn Trp Arg Val Gly Thr Gly Gly Trp Ser Pro Ala Asp Ser Asn
    50                  55                  60
Ala Gln Gln Trp Leu Gln Met Asp Leu Gly Asn Arg Val Glu Ile Thr
65                  70                  75                  80
Ala Val Ala Thr Gln Gly Arg Tyr Gly Ser Ser Asp Trp Val Thr Ser
                85                  90                  95
Tyr Ser Leu Met Phe Ser Asp Thr Gly Arg Asn Trp Lys Gln Tyr Lys
            100                 105                 110
Gln Glu Asp Ser Ile Trp Thr Phe Ala Gly Asn Met Asn Ala Asp Ser
        115                 120                 125
Val Val His His Lys Leu Leu His Ser Val Arg Ala Arg Phe Val Arg
    130                 135                 140
Phe Val Pro Leu Glu Trp Asn Pro Ser Gly Lys Ile Gly Met Arg Val
145                 150                 155                 160
Glu Val Tyr Gly Cys Ser Tyr Lys Ser Asp Val Ala Asp Phe Asp Gly
                165                 170                 175
Arg Ser Ser Leu Leu Tyr Arg Phe Asn Gln Lys Leu Met Ser Thr Leu
            180                 185                 190
Lys Asp Val Ile Ser Leu Lys Phe Lys Ser Met Gln Gly Asp Gly Val
        195                 200                 205
Leu Phe His Gly Glu Gly Gln Arg Gly Asp His Ile Thr Leu Glu Leu

210                 215                 220
Gln Lys Gly Arg Leu Ala Leu His Leu Asn Leu Gly Asp Ser Lys Ala
225                 230                 235                 240
Arg Leu Ser Ser Ser Leu Pro Ser Ala Thr Leu Gly Ser Leu Leu Asp
                245                 250                 255
Asp Gln His Trp His Ser Val Leu Ile Glu Arg Val Gly Lys Gln Val
            260                 265                 270
Asn Phe Thr Val Asp Lys His Thr Gln His Phe Arg Thr Lys Gly Glu
        275                 280                 285
Thr Asp Ala Leu Asp Ile Asp Tyr Glu Leu Ser Phe Gly Gly Ile Pro
    290                 295                 300
Val Pro Gly Lys Pro Gly Thr Phe Leu Lys Lys Asn Phe His Gly Cys
305                 310                 315                 320
Ile Glu Asn Leu Tyr Tyr Asn Gly Val Asn Ile Ile Asp Leu Ala Lys
                325                 330                 335
Arg Arg Lys His Gln Ile Tyr Thr Val Gly Asn Val Thr Phe Ser Cys
            340                 345                 350
Ser Glu Pro Gln Ile Val Pro Ile Thr Phe Val Asn Ser Ser Gly Ser
        355                 360                 365
Tyr Leu Leu Leu Pro Gly Thr Pro Gln Ile Asp Gly Leu Ser Val Ser
    370                 375                 380
Phe Gln Phe Arg Thr Trp Asn Lys Asp Gly Leu Leu Leu Ser Thr Glu
385                 390                 395                 400
Leu Ser Glu Gly Ser Gly Thr Leu Leu Leu Ser Leu Glu Gly Gly Ile
                405                 410                 415
Leu Arg Leu Val Ile Gln Lys Met Thr Glu Arg Val Ala Glu Ile Leu
            420                 425                 430
Thr Gly Ser Asn Leu Asn Asp Gly Leu Trp His Ser Val Ser Ile Asn
        435                 440                 445
Ala Arg Arg Asn Arg Ile Thr Leu Thr Leu Asp Asp Glu Ala Ala Pro
    450                 455                 460
Pro Ala Pro Asp Ser Thr Trp Val Gln Ile Tyr Ser Gly Asn Ser Tyr
465                 470                 475                 480
Tyr Phe Gly Gly Cys Pro Asp Asn Leu Thr Asp Ser Gln Cys Leu Asn
                485                 490                 495
Pro Ile Lys Ala Phe Gln Gly Cys Met Arg Leu Ile Phe Ile Asp Asn
            500                 505                 510
Gln Pro Lys Asp Leu Ile Ser Val Gln Gln Gly Ser Leu Gly Asn Phe
        515                 520                 525
Ser Asp Leu His Ile Asp Leu Cys Ser Ile Lys Asp Arg Cys Leu Pro
    530                 535                 540
Asn Tyr Cys Glu His Gly Gly Ser Cys Ser Gln Ser Trp Thr Thr Phe
545                 550                 555                 560
Tyr Cys Asn Cys Ser Asp Thr Ser Tyr Thr Gly Ala Thr Cys His Asn
                565                 570                 575
Ser Ile Tyr Glu Gln Ser Cys Glu Val Tyr Arg His Gln Gly Asn Thr
            580                 585                 590
Ala Gly Phe Phe Tyr Ile Asp Ser Asp Gly Ser Gly Pro Leu Gly Pro
        595                 600                 605
Leu Gln Val Tyr Cys Asn Ile Thr Glu Asp Lys Ile Trp Thr Ser Val
    610                 615                 620
Gln His Asn Asn Thr Glu Leu Thr Arg Val Arg Gly Ala Asn Pro Glu
625                 630                 635                 640
Lys Pro Tyr Ala Met Ala Leu Asp Tyr Gly Gly Ser Met Glu Gln Leu
                645                 650                 655
Glu Ala Val Ile Asp Gly Ser Glu His Cys Glu Gln Glu Val Ala Tyr
            660                 665                 670
His Cys Arg Arg Ser Arg Leu Leu Asn Thr Pro Asp Gly Thr Pro Phe
        675                 680                 685
Thr Trp Trp Ile Gly Arg Ser Asn Glu Arg His Pro Tyr Trp Gly Gly
    690                 695                 700
Ser Pro Pro Gly Val Gln Gln Cys Glu Cys Gly Leu Asp Glu Ser Cys
705                 710                 715                 720
Leu Asp Ile Gln His Phe Cys Asn Cys Asp Ala Asp Lys Asp Glu Trp
                725                 730                 735
Thr Asn Asp Thr Gly Phe Leu Ser Phe Lys Asp His Leu Pro Val Thr
            740                 745                 750
Gln Ile Val Ile Thr Asp Thr Asp Arg Ser Asn Ser Glu Ala Ala Trp

        755                 760                 765
Arg Ile Gly Pro Leu Arg Cys Tyr Gly Asp Arg Arg Phe Trp Asn Ala
    770                 775                 780
Val Ser Phe Tyr Thr Glu Ala Ser Tyr Leu His Phe Pro Thr Phe His
785                 790                 795                 800
Ala Glu Phe Ser Ala Asp Ile Ser Phe Phe Phe Lys Thr Thr Ala Leu
                805                 810                 815
Ser Gly Val Phe Leu Glu Asn Leu Gly Ile Lys Asp Phe Ile Arg Leu
            820                 825                 830
Glu Ile Ser Ser Pro Ser Glu Ile Thr Phe Ala Ile Asp Val Gly Asn
        835                 840                 845
Gly Pro Val Glu Leu Val Val Gln Ser Pro Ser Leu Leu Asn Asp Asn
    850                 855                 860
Gln Trp His Tyr Val Arg Ala Glu Arg Asn Leu Lys Glu Thr Ser Leu
865                 870                 875                 880
Gln Val Asp Asn Leu Pro Arg Ser Thr Arg Glu Thr Ser Glu Glu Gly
                885                 890                 895
His Phe Arg Leu Gln Leu Asn Ser Gln Leu Phe Val Gly Gly Thr Ser
            900                 905                 910
Ser Arg Gln Lys Gly Phe Leu Gly Cys Ile Arg Ser Leu His Leu Asn
        915                 920                 925
Gly Gln Lys Met Asp Leu Glu Glu Arg Ala Lys Val Thr Ser Gly Val
    930                 935                 940
Arg Pro Gly Cys Pro Gly His Cys Ser Ser Tyr Gly Ser Ile Cys His
945                 950                 955                 960
Asn Gly Gly Lys Cys Val Glu Lys His Asn Gly Tyr Leu Cys Asp Cys
                965                 970                 975
Thr Asn Ser Pro Tyr Glu Gly Pro Phe Cys Lys Lys Glu Val Ser Ala
            980                 985                 990
Val Phe Glu Ala Gly Thr Ser Val Thr Tyr Met Phe Gln Glu Pro Tyr
        995                 1000                1005
Pro Val Thr Lys Asn Ile Ser Leu Ser Ser Ser Ala Ile Tyr Thr Asp
    1010                1015                1020
Ser Ala Pro Ser Lys Glu Asn Ile Ala Leu Ser Phe Val Thr Thr Gln
1025                1030                1035                1040
Ala Pro Ser Leu Leu Leu Phe Ile Asn Ser Ser Ser Gln Asp Phe Val
                1045                1050                1055
Val Val Leu Leu Cys Lys Asn Gly Ser Leu Gln Val Arg Tyr His Leu
            1060                1065                1070
Asn Lys Glu Glu Thr His Val Phe Thr Ile Asp Ala Asp Asn Phe Ala
        1075                1080                1085
Asn Arg Arg Met His His Leu Lys Ile Asn Arg Glu Gly Arg Glu Leu
    1090                1095                1100
Thr Ile Gln Met Asp Gln Gln Leu Arg Leu Ser Tyr Asn Phe Ser Pro
1105                1110                1115                1120
Glu Val Glu Phe Arg Val Ile Arg Ser Leu Thr Leu Gly Lys Val Thr
                1125                1130                1135
Glu Asn Leu Gly Leu Asp Ser Glu Val Ala Lys Ala Asn Ala Met Gly
            1140                1145                1150
Phe Ala Gly Cys Met Ser Ser Val Gln Tyr Asn His Ile Ala Pro Leu
        1155                1160                1165
Lys Ala Ala Leu Arg His Ala Thr Val Ala Pro Val Thr Val His Gly
    1170                1175                1180
Thr Leu Thr Glu Ser Ser Cys Gly Phe Met Val Asp Ser Asp Val Asn
1185                1190                1195                1200
Ala Val Thr Thr Val His Ser Ser Ser Asp Pro Phe Gly Lys Thr Asp
                1205                1210                1215
Glu Arg Glu Pro Leu Thr Asn Ala Val Arg Ser Asp Ser Ala Val Ile
            1220                1225                1230
Gly Gly Val Ile Ala Val Val Ile Phe Ile Ile Phe Cys Ile Ile Gly
        1235                1240                1245
Ile Met Thr Arg Phe Leu Tyr Gln His Lys Gln Ser His Arg Thr Ser
    1250                1255                1260
Gln Met Lys Glu Lys Glu Tyr Pro Glu Asn Leu Asp Ser Ser Phe Arg
1265                1270                1275                1280
Asn Glu Ile Asp Leu Gln Asn Thr Val Ser Glu Cys Lys Arg Glu Tyr
                1285                1290                1295
Phe Ile

<210> 25
<211> 3528
<212> DNA
<213> homo sapiens

<400> 25
atgaatgctg acagcgtggt gcaccacaag ctattgcact cagtgagagc ccgatttgtt 60
cgctttgtgc ccctggaatg gaatcccagt gggaagattg gcatgagagt cgaggtctac 120
ggatgttcct ataaatcaga cgttgctgac tttgatggcc gaagctcact tctgtacagg 180
ttcaatcaga agttgatgag tactctcaaa gatgtgatct ccctgaagtt caagagcatg 240
caaggagatg gggtcctgtt ccatggagaa ggtcagcgtg gagaccacat caccttggaa 300
ctccagaagg ggaggctcgc cctacacctc aatttgggtg acagcaaagc gcggctcagc 360
agcagcttgc cctctgccac cctgggcagc ctcctggatg accagcactg gcactcggtc 420
ctcattgagc gggtgggcaa gcaggtgaac ttcacggtgg acaagcacac acagcacttc 480
cgcaccaagg gcgagacgga tgccttagac attgactatg agcttagttt tggaggaatt 540
ccagtaccag gaaaacctgg gaccttttta aagaaaaact tccatggatg catcgaaaac 600
ctttactaca atggagtaaa cataattgac ctggctaaga gacgaaagca tcagatctat 660
actgtgggca atgtcacttt ttcctgctcc gaaccacaga ttgtgcccat cacatttgtc 720
aactccagcg gcagctattt gctgctgccc ggcacccccc aaattgatgg gctctcagtg 780
agtttccagt ttcgaacatg gaacaaggat ggtctgcttc tgtccacaga gctgtctgag 840
ggctcgggaa ccctgctgct gagcctggag ggtggaatcc tgagactcgt gattcagaaa 900
atgacagaac gcgtagctga aatcctcaca ggcagcaact tgaatgatgg cctgtggcac 960
tcggttagca tcaacgccag gaggaaccgc atcacgctca ctctggatga tgaagcagca 1020
cccccggctc cagacagcac ttgggtgcag atttattctg gaaatagcta ctattttgga 1080
gggtgccccg acaatctcac cgattcccaa tgtttaaatc ccattaaggc tttccaaggc 1140
tgcatgaggc tcatctttat tgataaccag cccaaggacc tcatttcagt tcagcaaggt 1200
tccctgggga attttagtga tttacacatt gatctgtgta gcatcaaaga caggtgtttg 1260
ccaaactact gtgaacatgg aggaagctgc tcccagtcct ggactacctt ctattgtaac 1320
tgcagtgaca caagttacac tggtgccacc tgccacaact ccatctacga gcaatcctgc 1380
gaggtgtaca ggcaccaggg gaatacagcc ggcttcttct acatcgactc agatggcagc 1440
ggcccactgg gacctctcca ggtgtactgc aatatcactg aggacaagat ctggacatca 1500
gtgcagcaca acaatacaga gctgacccga gtgcggggcg ctaaccctga gaagccctat 1560
gccatggcct tggactacgg gggcagcatg gaacagctgg aggccgtgat cgacggctct 1620
gagcactgtg agcaggaggt ggcctaccac tgcaggaggt cccgcctgct caacacgccg 1680
gatggaacac catttacctg gtggattggg cggtccaatg aaaggcaccc ttactgggga 1740
ggttcccctc ctggggtcca gcagtgtgag tgtggcctag acgagagctg cctggacatt 1800
cagcactttt gcaattgcga cgctgacaag gatgaatgga caaatgatac tggctttctt 1860
tccttcaaag accacttgcc tgtcactcag atagttatca ctgataccga cagatcaaac 1920
tcagaagccg cttggagaat tggtcccttg cgttgctatg gtgaccgacg cttctggaac 1980
gccgtctcat tttatacaga agcctcttac ctccactttc ctaccttcca tgcggaattc 2040
agtgccgata tttccttctt ttttaaaacc acagcattat ccggagtttt cctagaaaat 2100
cttggcatta aagacttcat tcgactcgaa ataagctctc cttcagagat cacctttgcc 2160
atcgatgttg ggaatggtcc tgtggagctt gtagtccagt ctccttctct tctgaatgac 2220
aaccaatggc actatgtccg ggctgagagg aacctcaagg agacctccct gcaggtggac 2280
aaccttccaa ggagcaccag ggagacgtcg gaggagggcc attttcgact gcagctgaac 2340
agccagttgt ttgtaggggg aacgtcatcc agacagaaag gcttcctagg atgcattcgc 2400
tccttacact tgaatggaca gaaaatggac ctggaagaga gggcaaaggt cacatctgga 2460
gtcaggccag gctgccccgg ccactgcagc agctacggca gcatctgcca caacgggggc 2520
aagtgtgtgg agaagcacaa tggctacctg tgtgattgca ccaattcacc ttatgaaggg 2580
cccttttgca aaaaagaggt ttctgctgtt tttgaggctg gcacgtcggt tacttacatg 2640
tttcaagaac cctatcctgt gaccaagaat ataagcctct catcctcagc tatttacaca 2700
gattcagctc catccaagga aaacattgca cttagctttg tgacaaccca ggcacccagt 2760
cttttgctct ttatcaattc ttcttctcag gacttcgtgg ttgttctgct ctgcaagaat 2820
ggaagcttac aggttcgcta tcacctaaac aaggaagaaa cccatgtatt caccattgat 2880
gcagataact ttgctaacag aaggatgcac cacttgaaga ttaaccgaga gggaagagag 2940
cttaccattc agatggacca gcaacttcga ctcagttata acttctctcc ggaagtagag 3000
ttcagggtta taaggtcact caccttgggc aaagtcacag agaatcttgg tttggattct 3060
gaagttgcta aagcaaatgc catgggtttt gctggatgca tgtcttccgt ccagtacaac 3120
cacatagcac cactgaaggc tgccctgcgc catgccactg tcgcgcctgt gactgtccat 3180
gggaccttga cggaatccag ctgtggcttc atggtggact cagatgtgaa tgcagtgacc 3240
acggtgcatt cttcatcaga tccttttggg aagacagatg agcgggaacc actcacaaat 3300
gctgttcgaa gtgattcggc agtcatcgga ggggtgatag cagtggtgat attcatcatc 3360
ttctgtatca tcggcatcat gacccggttc ctctaccagc acaagcagtc acatcgtacg 3420
agccagatga aggagaagga atatccagaa aatttggaca gttccttcag aaatgaaatt 3480
gacttgcaaa acacagtgag cgagtgtaaa cgggaatatt tcatctga 3528

<210> 26
<211> 1175
<212> PRT
<213> homo sapiens

<400> 26
Met Asn Ala Asp Ser Val Val His His Lys Leu Leu His Ser Val Arg
1               5                   10                  15
Ala Arg Phe Val Arg Phe Val Pro Leu Glu Trp Asn Pro Ser Gly Lys
            20                  25                  30
Ile Gly Met Arg Val Glu Val Tyr Gly Cys Ser Tyr Lys Ser Asp Val
        35                  40                  45
Ala Asp Phe Asp Gly Arg Ser Ser Leu Leu Tyr Arg Phe Asn Gln Lys
    50                  55                  60
Leu Met Ser Thr Leu Lys Asp Val Ile Ser Leu Lys Phe Lys Ser Met
65                  70                  75                  80
Gln Gly Asp Gly Val Leu Phe His Gly Glu Gly Gln Arg Gly Asp His
                85                  90                  95
Ile Thr Leu Glu Leu Gln Lys Gly Arg Leu Ala Leu His Leu Asn Leu
            100                 105                 110
Gly Asp Ser Lys Ala Arg Leu Ser Ser Ser Leu Pro Ser Ala Thr Leu
        115                 120                 125
Gly Ser Leu Leu Asp Asp Gln His Trp His Ser Val Leu Ile Glu Arg
    130                 135                 140
Val Gly Lys Gln Val Asn Phe Thr Val Asp Lys His Thr Gln His Phe
145                 150                 155                 160
Arg Thr Lys Gly Glu Thr Asp Ala Leu Asp Ile Asp Tyr Glu Leu Ser
                165                 170                 175
Phe Gly Gly Ile Pro Val Pro Gly Lys Pro Gly Thr Phe Leu Lys Lys
            180                 185                 190
Asn Phe His Gly Cys Ile Glu Asn Leu Tyr Tyr Asn Gly Val Asn Ile
        195                 200                 205
Ile Asp Leu Ala Lys Arg Arg Lys His Gln Ile Tyr Thr Val Gly Asn
    210                 215                 220
Val Thr Phe Ser Cys Ser Glu Pro Gln Ile Val Pro Ile Thr Phe Val
225                 230                 235                 240
Asn Ser Ser Gly Ser Tyr Leu Leu Leu Pro Gly Thr Pro Gln Ile Asp
                245                 250                 255
Gly Leu Ser Val Ser Phe Gln Phe Arg Thr Trp Asn Lys Asp Gly Leu
            260                 265                 270
Leu Leu Ser Thr Glu Leu Ser Glu Gly Ser Gly Thr Leu Leu Leu Ser
        275                 280                 285
Leu Glu Gly Gly Ile Leu Arg Leu Val Ile Gln Lys Met Thr Glu Arg
    290                 295                 300
Val Ala Glu Ile Leu Thr Gly Ser Asn Leu Asn Asp Gly Leu Trp His
305                 310                 315                 320
Ser Val Ser Ile Asn Ala Arg Arg Asn Arg Ile Thr Leu Thr Leu Asp
                325                 330                 335
Asp Glu Ala Ala Pro Pro Ala Pro Asp Ser Thr Trp Val Gln Ile Tyr
            340                 345                 350
Ser Gly Asn Ser Tyr Tyr Phe Gly Gly Cys Pro Asp Asn Leu Thr Asp
        355                 360                 365
Ser Gln Cys Leu Asn Pro Ile Lys Ala Phe Gln Gly Cys Met Arg Leu
    370                 375                 380
Ile Phe Ile Asp Asn Gln Pro Lys Asp Leu Ile Ser Val Gln Gln Gly
385                 390                 395                 400
Ser Leu Gly Asn Phe Ser Asp Leu His Ile Asp Leu Cys Ser Ile Lys
                405                 410                 415
Asp Arg Cys Leu Pro Asn Tyr Cys Glu His Gly Gly Ser Cys Ser Gln
            420                 425                 430
Ser Trp Thr Thr Phe Tyr Cys Asn Cys Ser Asp Thr Ser Tyr Thr Gly
        435                 440                 445
Ala Thr Cys His Asn Ser Ile Tyr Glu Gln Ser Cys Glu Val Tyr Arg
    450                 455                 460
His Gln Gly Asn Thr Ala Gly Phe Phe Tyr Ile Asp Ser Asp Gly Ser
465                 470                 475                 480
Gly Pro Leu Gly Pro Leu Gln Val Tyr Cys Asn Ile Thr Glu Asp Lys

                485                 490                 495
Ile Trp Thr Ser Val Gln His Asn Asn Thr Glu Leu Thr Arg Val Arg
            500                 505                 510
Gly Ala Asn Pro Glu Lys Pro Tyr Ala Met Ala Leu Asp Tyr Gly Gly
        515                 520                 525
Ser Met Glu Gln Leu Glu Ala Val Ile Asp Gly Ser Glu His Cys Glu
    530                 535                 540
Gln Glu Val Ala Tyr His Cys Arg Arg Ser Arg Leu Leu Asn Thr Pro
545                 550                 555                 560
Asp Gly Thr Pro Phe Thr Trp Trp Ile Gly Arg Ser Asn Glu Arg His
                565                 570                 575
Pro Tyr Trp Gly Gly Ser Pro Pro Gly Val Gln Gln Cys Glu Cys Gly
            580                 585                 590
Leu Asp Glu Ser Cys Leu Asp Ile Gln His Phe Cys Asn Cys Asp Ala
        595                 600                 605
Asp Lys Asp Glu Trp Thr Asn Asp Thr Gly Phe Leu Ser Phe Lys Asp
    610                 615                 620
His Leu Pro Val Thr Gln Ile Val Ile Thr Asp Thr Asp Arg Ser Asn
625                 630                 635                 640
Ser Glu Ala Ala Trp Arg Ile Gly Pro Leu Arg Cys Tyr Gly Asp Arg
                645                 650                 655
Arg Phe Trp Asn Ala Val Ser Phe Tyr Thr Glu Ala Ser Tyr Leu His
            660                 665                 670
Phe Pro Thr Phe His Ala Glu Phe Ser Ala Asp Ile Ser Phe Phe Phe
        675                 680                 685
Lys Thr Thr Ala Leu Ser Gly Val Phe Leu Glu Asn Leu Gly Ile Lys
    690                 695                 700
Asp Phe Ile Arg Leu Glu Ile Ser Ser Pro Ser Glu Ile Thr Phe Ala
705                 710                 715                 720
Ile Asp Val Gly Asn Gly Pro Val Glu Leu Val Val Gln Ser Pro Ser
                725                 730                 735
Leu Leu Asn Asp Asn Gln Trp His Tyr Val Arg Ala Glu Arg Asn Leu
            740                 745                 750
Lys Glu Thr Ser Leu Gln Val Asp Asn Leu Pro Arg Ser Thr Arg Glu
        755                 760                 765
Thr Ser Glu Glu Gly His Phe Arg Leu Gln Leu Asn Ser Gln Leu Phe
    770                 775                 780
Val Gly Gly Thr Ser Ser Arg Gln Lys Gly Phe Leu Gly Cys Ile Arg
785                 790                 795                 800
Ser Leu His Leu Asn Gly Gln Lys Met Asp Leu Glu Glu Arg Ala Lys
                805                 810                 815
Val Thr Ser Gly Val Arg Pro Gly Cys Pro Gly His Cys Ser Ser Tyr
            820                 825                 830
Gly Ser Ile Cys His Asn Gly Gly Lys Cys Val Glu Lys His Asn Gly
        835                 840                 845
Tyr Leu Cys Asp Cys Thr Asn Ser Pro Tyr Glu Gly Pro Phe Cys Lys
    850                 855                 860
Lys Glu Val Ser Ala Val Phe Glu Ala Gly Thr Ser Val Thr Tyr Met
865                 870                 875                 880
Phe Gln Glu Pro Tyr Pro Val Thr Lys Asn Ile Ser Leu Ser Ser Ser
                885                 890                 895
Ala Ile Tyr Thr Asp Ser Ala Pro Ser Lys Glu Asn Ile Ala Leu Ser
            900                 905                 910
Phe Val Thr Thr Gln Ala Pro Ser Leu Leu Leu Phe Ile Asn Ser Ser
        915                 920                 925
Ser Gln Asp Phe Val Val Val Leu Leu Cys Lys Asn Gly Ser Leu Gln
    930                 935                 940
Val Arg Tyr His Leu Asn Lys Glu Glu Thr His Val Phe Thr Ile Asp
945                 950                 955                 960
Ala Asp Asn Phe Ala Asn Arg Arg Met His His Leu Lys Ile Asn Arg
                965                 970                 975
Glu Gly Arg Glu Leu Thr Ile Gln Met Asp Gln Gln Leu Arg Leu Ser
            980                 985                 990
Tyr Asn Phe Ser Pro Glu Val Glu Phe Arg Val Ile Arg Ser Leu Thr
        995                 1000                1005
Leu Gly Lys Val Thr Glu Asn Leu Gly Leu Asp Ser Glu Val Ala Lys
    1010                1015                1020
Ala Asn Ala Met Gly Phe Ala Gly Cys Met Ser Ser Val Gln Tyr Asn

1025                1030                1035                1040
His Ile Ala Pro Leu Lys Ala Ala Leu Arg His Ala Thr Val Ala Pro
            1045                1050                1055
Val Thr Val His Gly Thr Leu Thr Glu Ser Ser Cys Gly Phe Met Val
        1060                1065                1070
Asp Ser Asp Val Asn Ala Val Thr Thr Val His Ser Ser Ser Asp Pro
    1075                1080                1085
Phe Gly Lys Thr Asp Glu Arg Glu Pro Leu Thr Asn Ala Val Arg Ser
    1090                1095                1100
Asp Ser Ala Val Ile Gly Gly Val Ile Ala Val Val Ile Phe Ile Ile
1105                1110                1115                1120
Phe Cys Ile Ile Gly Ile Met Thr Arg Phe Leu Tyr Gln His Lys Gln
            1125                1130                1135
Ser His Arg Thr Ser Gln Met Lys Glu Lys Glu Tyr Pro Glu Asn Leu
        1140                1145                1150
Asp Ser Ser Phe Arg Asn Glu Ile Asp Leu Gln Asn Thr Val Ser Glu
    1155                1160                1165
Cys Lys Arg Glu Tyr Phe Ile
    1170                1175

<210> 27
<211> 4869
<212> DNA
<213> homo sapiens

<400> 27
   attgggtttg gatttgcacc gttaaggagg ggggaagaga aggaagaggc gggcgaggaa 60
   ggcgagtcca gctagcggct gttgcgggga ccgtagcccc agctgcagct ccgaagaatc 120
   ccccgccacg gtttcggtgg agcgtctggg cacgggatgg agtgaaagag cgagtgcctc 180
   tccaagcggg ggtgggaggg ggtcaggctg tgcagaggag agagacagcg agaagaagcc 240
   gcggctggct actgcgaatt tgggattcga ttgggaggga ccgctcactc gggggaaatg 300
   gattctttac cacggctgac cagcgttttg actttgctgt tctctggctt gtggcattta 360
   ggattaacag cgacaaactg tgaggagtag atgcagtaac acatggaaac cgggagtacc 420
   tgaccttgtt cagagcaggt ggttaataaa tgatggtagt taatttctca acttctcttc 480
   ttatggaatg tttcttgcta atagaatctg gtctctctcc ctctgtcctc ctatcattat 540
   agacaactgt gatgatccac tagcatccct gctctctcca atggcttttt ccagttcctc 600
   agacctcact ggcactcaca gcccagctca actcaactgg agagttggaa ctggcggttg 660
   gtccccagca gattccaatg ctcaacagtg gctccagatg gacctgggaa acagagtaga 720
   gattacagca gtggccacgc agggaagata cggaagctct gactgggtga cgagttacag 780
   cctgatgttc agtgacacag gacgcaactg gaaacagtac aaacaagaag acagcatctg 840
   gacctttgca ggaaacatga atgctgacag cgtggtgcac cacaagctat tgcactcagt 900
   gagagcccga tttgttcgct ttgtgcccct ggaatggaat cccagtggga agattggcat 960
   gagagtcgag gtctacggat gttcctataa atcagacgtt gctgactttg atggccgaag 1020
   ctcacttctg tacaggttca atcagaagtt gatgagtact ctcaaagatg tgatctccct 1080
   gaagttcaag agcatgcaag gagatggggt cctgttccat ggagaaggtc agcgtggaga 1140
   ccacatcacc ttggaactcc agaaggggag gctcgcccta cacctcaatt tgggtgacag 1200
   caaagcgcgg ctcagcagca gcttgccctc tgccaccctg ggcagcctcc tggatgacca 1260
   gcactggcac tcggtcctca ttgagcgggt gggcaagcag gtgaacttca cggtggacaa 1320
   gcacacacag cacttccgca ccaagggcga gacggatgcc ttagacattg actatgagct 1380
   tagttttgga ggaattccag taccaggaaa acctgggacc tttttaaaga aaaacttcca 1440
   tggatgcatc gaaaaccttt actacaatgg agtaaacata attgacctgg ctaagagacg 1500
   aaagcatcag atctatactg tgggcaatgt cactttttcc tgctccgaac cacagattgt 1560
   gcccatcaca tttgtcaact ccagcggcag ctatttgctg ctgcccggca ccccccaaat 1620
   tgatgggctc tcagtgagtt tccagtttcg aacatggaac aaggatggtc tgcttctgtc 1680
   cacagagctg tctgagggct cgggaaccct gctgctgagc ctggagggtg gaatcctgag 1740
   actcgtgatt cagaaaatga cagaacgcgt agctgaaatc ctcacaggca gcaacttgaa 1800
   tgatggcctg tggcactcgg ttagcatcaa cgccaggagg aaccgcatca cgctcactct 1860
   ggatgatgaa gcagcacccc cggctccaga cagcacttgg gtgcagattt attctggaaa 1920
   tagctactat tttggagggt gccccgacaa tctcaccgat tcccaatgtt taaatcccat 1980
   taaggctttc caaggctgca tgaggctcat ctttattgat aaccagccca aggacctcat 2040
   ttcagttcag caaggttccc tggggaattt tagtgattta cacattgatc tgtgtagcat 2100
   caaagacagg tgtttgccaa actactgtga acatggagga agctgctccc agtcctggac 2160
   taccttctat tgtaactgca gtgacacaag ttacactggt gccacctgcc acaactccat 2220
   ctacgagcaa tcctgcgagg tgtacaggca ccaggggaat acagccggct tcttctacat 2280
   cgactcagat ggcagcggcc cactgggacc tctccaggtg tactgcaata tcactgagga 2340
   caagatctgg acatcagtgc agcacaacaa tacagagctg acccgagtgc ggggcgctaa 2400
   ccctgagaag ccctatgcca tggccttgga ctacgggggc agcatggaac agctggaggc 2460
   cgtgatcgac ggctctgagc actgtgagca ggaggtggcc taccactgca ggaggtcccg 2520
   cctgctcaac acgccggatg gaacaccatt tacctggtgg attgggcggt ccaatgaaag 2580
   gcacccttac tggggaggtt cccctcctgg ggtccagcag tgtgagtgtg gcctagacga 2640
   gagctgcctg gacattcagc acttttgcaa ttgcgacgct gacaaggatg aatggacaaa 2700
   tgatactggc tttctttcct tcaaagacca cttgcctgtc actcagatag ttatcactga 2760
   taccgacaga tcaaactcag aagccgcttg gagaattggt cccttgcgtt gctatggtga 2820
   ccgacgcttc tggaacgccg tctcatttta tacagaagcc tcttacctcc actttcctac 2880
   cttccatgcg gaattcagtg ccgatatttc cttctttttt aaaaccacag cattatccgg 2940
   agttttccta gaaaatcttg gcattaaaga cttcattcga ctcgaaataa gctctccttc 3000
   agagatcacc tttgccatcg atgttgggaa tggtcctgtg gagcttgtag tccagtctcc 3060
   ttctcttctg aatgacaacc aatggcacta tgtccgggct gagaggaacc tcaaggagac 3120
   ctccctgcag gtggacaacc ttccaaggag caccagggag acgtcggagg agggccattt 3180
   tcgactgcag ctgaacagcc agttgtttgt agggggaacg tcatccagac agaaaggctt 3240
   cctaggatgc attcgctcct tacacttgaa tggacagaaa atggacctgg aagagagggc 3300
   aaaggtcaca tctggagtca ggccaggctg ccccggccac tgcagcagct acggcagcat 3360
   ctgccacaac gggggcaagt gtgtggagaa gcacaatggc tacctgtgtg attgcaccaa 3420
   ttcaccttat gaagggccct tttgcaaaaa agaggtttct gctgtttttg aggctggcac 3480
   gtcggttact tacatgtttc aagaacccta tcctgtgacc aagaatataa gcctctcatc 3540
   ctcagctatt tacacagatt cagctccatc caaggaaaac attgcactta gctttgtgac 3600
   aacccaggca cccagtcttt tgctctttat caattcttct tctcaggact tcgtggttgt 3660
   tctgctctgc aagaatggaa gcttacaggt tcgctatcac ctaaacaagg aagaaaccca 3720
   tgtattcacc attgatgcag ataactttgc taacagaagg atgcaccact tgaagattaa 3780
   ccgagaggga agagagctta ccattcagat ggaccagcaa cttcgactca gttataactt 3840
   ctctccggaa gtagagttca gggttataag gtcactcacc ttgggcaaag tcacagagaa 3900
   tcttggtttg gattctgaag ttgctaaagc aaatgccatg ggttttgctg gatgcatgtc 3960
   ttccgtccag tacaaccaca tagcaccact gaaggctgcc ctgcgccatg ccactgtcgc 4020
   gcctgtgact gtccatggga ccttgacgga atccagctgt ggcttcatgg tggactcaga 4080
   tgtgaatgca gtgaccacgg tgcattcttc atcagatcct tttgggaaga cagatgagcg 4140
   ggaaccactc acaaatgctg ttcgaagtga ttcggcagtc atcggagggg tgatagcagt 4200
   ggtgatattc atcatcttct gtatcatcgg catcatgacc cggttcctct accagcacaa 4260
   gcagtcacat cgtacgagcc agatgaagga gaaggaatat ccagaaaatt tggacagttc 4320
   cttcagaaat gaaattgact tgcaaaacac agtgagcgag tgtaaacggg aatatttcat 4380
   ctgagaaact gcagggttcc tactactctt ttttcttgtt gttcaattat ctcctccccc 4440
   tcttctctcc tgtcttttga tttggtcatt ctctttattt tctgcttgcc atgtcttttc 4500
   tggaacatac ttgcatccac cacagcatca attcccttga tccagcccaa gagaccaggc 4560
   agccatggcc actgccttcc tctctgatga acctatcggg tgaaaacgac cactcaagag 4620
   actgacttcg ccattcaaga caaggaagag acacatgtgt gcactcctgc atgttcagtt 4680
   ctgtacttcc agtttctaaa atgcactgtt cagttttcca accacttggt ggttcaggct 4740
   tgctttgaac ctgagctctt aggcacatga cggtcattcc tgacatcctc cccagctcaa 4800
   gtctattctt accatagaac ccagggcagg gagagaagaa cctagaggcc tggtttgctt 4860
   tggtggcat 4869